(19) Europäisches Patentamt / European Patent Office / Office européen des brevets

(11) **EP 3 538 653 B1**

(12) # EUROPEAN PATENT SPECIFICATION

(45) Date of publication and mention of the grant of the patent:
**28.07.2021 Bulletin 2021/30**

(21) Application number: **17817014.8**

(22) Date of filing: **08.11.2017**

(51) Int Cl.:
***C12N 15/10*** *(2006.01)* ***C12Q 1/6823*** *(2018.01)*
***C40B 40/06*** *(2006.01)*

(86) International application number:
**PCT/GB2017/053360**

(87) International publication number:
**WO 2018/087539 (17.05.2018 Gazette 2018/20)**

(54) **TAGLESS ENCODED CHEMICAL LIBRARY**

ETIKETTENLOSE CODIERTE CHEMISCHE BIBLIOTHEK

BIBLIOTHÈQUE CHIMIQUE CODÉE SANS ÉTIQUETTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2016 GB 201618820**
**19.06.2017 GB 201709718**

(43) Date of publication of application:
**18.09.2019 Bulletin 2019/38**

(73) Proprietor: **Nanna Therapeutics Limited**
**Cambridge**
**Cambridgeshire CB1 3LQ (GB)**

(72) Inventors:
- **WILLIAMS, David Hugh**
  **Melbourn**
  **Cambridgeshire SG8 6BB (GB)**
- **MANDAL, Ipshita**
  **Rosemary Lane**
  **Cambridge CB1 3LQ (GB)**
- **WOOD, Stuart Robert**
  **Cambridge CB24 3BF (GB)**
- **BRATTON, Daniel**
  **Sawston**
  **Cambridgeshire CB22 3BN (GB)**

(74) Representative: **Hutchins, Michael Richard et al**
**Schlich**
**9 St Catherine's Road**
**Littlehampton, West Sussex BN17 5HS (GB)**

(56) References cited:
**WO-A1-2004/087308 WO-A1-2012/019765**
**WO-A1-2016/145409**

- **NACHIKET SHEMBEKAR ET AL: "Droplet-based microfluidics in drug discovery, transcriptomics and high-throughput molecular genetics", LAB ON A CHIP, vol. 16, no. 8, 18 March 2016 (2016-03-18) , pages 1314-1331, XP055451515, ISSN: 1473-0197, DOI: 10.1039/C6LC00249H**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the Invention

The present invention relates to encoded chemical libraries, and in particular to nucleic acid encoded libraries containing tagless chemical structures spatially associated with encoding tags. The invention also relates to methods for nucleic acid-templated chemical library generation, methods for screening the encoded libraries and to compositions for preparing and screening the encoded libraries.

Background to the Invention

Drug discovery typically involves the assembly of large libraries of chemical compounds followed by an assay or screen in which the compounds are added individually to microwells that contain a target to identify "hits" which display a desired activity on the target (e.g. enzymatic activity or displacement of a label). This process is known as high-throughput screening (HTS). Although it can be automated using robotic equipment to test millions of chemicals, it is both laborious and expensive.

There is therefore a fundamental problem stemming from the fact that increased library size increases the screening burden: due to the discrete nature of screening assays, screening time and cost scale approximately linearly with library size. This has imposed severe practical constraints on the size of the chemical libraries screenable using such approaches: HTS is typically applied to libraries containing $10^3$-$10^6$ members.

This problem has been addressed by the development of screening techniques based on selection (e.g. panning techniques). Here, all of the compounds in the library are simultaneously tested for their ability to interact with a target of interest in a one-pot format. In such assays, the time and cost of the screening step is independent of library size, and so the assay can be applied to relatively large libraries. Libraries containing up to $10^{12}$ members have been screened using such approaches.

The problem has also been addressed by the development of microdroplet-based libraries which can be processed by microfluidic techniques to increase throughput by several orders of magnitude and which are compatible with cell-based phenotypic screens (see e.g. Clausell-Tormos et al. (2008) Chemistry & Biology 15: 427-437).

However, both selection-based assays and microdroplet-based screens require that the identity of the selected chemical compounds (i.e. the "hits") be readily determined: libraries that are screenable but not decodable are not useful.

A solution to this problem was first proposed in 1992 by Brenner and Lerner (Brenner and Lerner (1992) Proc. Natl. Acad. Sci. USA. 89: 5381-5383), and is based on the generation of DNA encoded chemical libraries (DECLs). In a DECL, each compound is linked (tagged) with a DNA sequence which corresponds to its structure or reaction history, thereby serving as a unique identifier of that particular compound (i.e. the DNA tag "encodes" that compound, so serving as a molecular "barcode").

The compounds can be tagged in various different ways, and it is also possible to use the DNA tag not just to encode a specific chemical structure ("DNA recording"), but also as a template which directs its synthesis ("DNA templating"). The technology has been recently reviewed by Mannocci et al. (2011) Chem. Commun., 47: 12747-12753; Kleiner et al. (2011) Chem Soc Rev. 40(12): 5707-5717; and Mullard (2016) Nature 530: 367-369.

DECL technology is now well-established within the pharmaceutical industry: in 2007, GSK acquired one of the firms that pioneered DECLs, Praecis Pharmaceuticals, for US$55 million, while other top-ten Pharma have started their own in-house DNA-encoded-library programmes. Other biotech companies, including X-Chem, Vipergen, Ensemble Therapeutics and Philochem are also actively developing and exploiting DECL technology.

However, the utility of current DECLs are currently limited by problems associated with the presence of the tag. For example, the encoding tag may: (a) chemically or sterically interfere with the access of the tagged compounds to molecular binding sites on targets of interest, so limiting the number and/or type of hits recovered; (b) limit cellular permeability and/or diffusion, effectively preventing cellular uptake and excluding the use of cell-based phenotypic screens which rely on access to the cytoplasm; (c) limit the extent to which the chemical compounds can be chemically modified after tagging (certain reactions being chemically incompatible with the tag); and (d) limit the usefulness of structure-activity analyses, since such analyses are confounded by the potential impact of the tag itself on activity. Yet further limitations arise from the fact that the current methods yield mixed pools of compounds comprising a multitude of compounds at low concentration. This leads to a need for deconvolution of the active from the pool, while the low concentrations severely constrain the format and nature of the applicable screens: homogeneous cell-based phenotypic screening is not possible.

Shembekar et al. (2016) Lab Chip, 2016, 16: 1314-1331 descibes the application of droplet-based microfluidics in drug discovery, transcriptomics and high-throughput molecular genetics. Shembekar *et al.* also considers the use of encoding tags that can be released from a defined chemical compound under defined conditions (e.g. exposure to uv radiation) within the confines of a droplet. This allows maintenance of the spacial association of tag and compound so

that the compound can be identified by the tag after release.

There is therefore a need for HTS techniques which permit the screening of decodable chemical libraries and which address the foregoing problems.

## Summary of the Invention

According to a first aspect of the present invention, there is provided a method for screening an encoded chemical library, which library comprises a number *n* of clonal populations of different chemical structures each releasably linked to an encoding tag by a cleavable linker via a bead, and each clonal population being confined to *n* discrete library microcompartments, the method comprising the steps of: (a) providing said library of tagged chemical structures; (b) placing each of the library microcompartments are placed into discrete screening microdroplets, which microdroplets comprise a gelling system; (c) releasing each chemical structure from its tag to produce a plurality of free, tagless chemical structures (TCSs); (d) phenotypically screening the TCSs by contacting them with an assay system comprising a live target cell under conditions whereby a spatial association between each TCS and its tag is maintained, to produce a plurality of different screened TCSs each spatially associated with its tag; and (e) identifying a screened TCS by decoding a tag that is spatially associated therewith wherein the gelling system of the microdroplets is gelled prior to the identifying step (e) such that the library microcompartment becomes fixed within the gelled microdroplet.

The screening can be carried out at any concentration of TCSs. Preferably the concentration of TCSs facilitates fragment-based screening and may, for example, fall in the nM to mM range.

The methods of the invention therefore permit the screening of libraries of tagless encoded chemical structures, so obviating the problems associated with the presence of an encoding tag.

### *Tags*

Any encoding tag may be used according to the invention provided that it contains information (for example, in the form of chemical and/or optical properties/characteristics) which uniquely identifies its cognate chemical structure (or its reaction history), thereby serving as a unique identifier of that particular chemical structure. Thus, the tag "encodes" a particular chemical structure and serves as a molecular "barcode". In preferred embodiments the tag is a nucleic acid (for example DNA) tag in which the information is encoded in the sequence of the nucleic acid. However, other tags may be used, including non-DNA tags, non-RNA tags, modified nucleic acid tags, peptide tags, light-based barcodes (e.g. quantum dots) and RFID tags.

As explained above, the chemical structures can be tagged in various different ways, and it is also possible to use the DNA tag not just to encode a specific chemical structure ("DNA recording"), but also as a template which directs its synthesis ("DNA templating" - see below). The technology has been recently reviewed by Mannocci et al. (2011) Chem. Commun., 47: 12747-12753; Kleiner et al. (2011) Chem Soc Rev. 40(12): 5707-5717; and Mullard (2016) Nature 530: 367-369.

In certain embodiments, a split-and-pool tagging technique is employed (see e.g. Mannocci et al. (2011) Chem. Commun., 47: 12747-12753, and in particular Figure 3 thereof).

The identifying step (e) comprises the step of decoding the encoding tag, and thus is selected according to the nature of the encoding tag. In the case of nucleic acid tags, the identifying step comprises sequencing the nucleic acid (e.g. DNA).

The chemical structures may be small molecules (as herein defined). In certain embodiments, the structures are comprised of a number of linked substructures. In other embodiments, for example in screens for non-drug applications, the chemical structures may be large molecules (as herein defined).

### *Linkers*

The chemical structures may be (directly or indirectly) releasably linked to the encoding tag by a cleavable linker. In such embodiments, the cleavable linker may comprise a linker selected from: enzymatically cleavable linkers; nucleophile/base-sensitive linkers; reduction sensitive linkers; photocleavable linkers; electrophile/acid-sensitive linkers; metal-assisted cleavage-sensitive linkers; oxidation-sensitive linkers; and combinations of two or more of the foregoing.

The chemical structures may be (directly or indirectly) releasably linked to the encoding tag by nucleic acid hybridization. In such embodiments, the chemical structures may also be (directly or indirectly) releasably linked to the encoding tag by a cleavable linker, as described above.

In preferred embodiments, the cleavable linker may comprise RNA and in such embodiments step (c) may comprise contacting the tagged chemical structures with an RNAse.

In other embodiments, the cleavable linker may comprise a peptide and in such embodiments step (c) may comprise contacting the tagged chemical structures with a peptidase.

In other embodiments, the cleavable linker may comprise DNA and in such embodiments step (c) may comprise contacting the tagged chemical structures with a site-specific endonuclease.

The chemical structures may be releasably linked to the encoding tag by nucleic acid which: (a) hybridizes to the nucleic acid of the encoding tag; and (b) is coupled to the chemical structure. In such embodiments, the hybridizing nucleic acid may be coupled to the chemical structure by a cleavable linker as defined above. Here, the hybridizing nucleic acid is preferably RNA, in which case step (c) may comprise contacting the tagged chemical structures with an RNAse.

In the methods of the invention, step (c) may comprise dehybridization, for example melting, of nucleic acid coupled to the chemical structure and hybridized to the nucleic acid of the encoding tag.

*Library microcompartments*

The encoded chemical library of step (a) may comprise a number *n* of clonal populations of tagged chemical structures, each clonal population being confined to *n* discrete library microcompartments. In such embodiments: (a) $n > 10^3$; or (b) $n > 10^4$; or (c) $n > 10^5$; or (d) $n > 10^6$; or (e) $n > 10^7$; or (f) $n > 10^8$; or (g) $n > 10^9$; or (h) $n > 10^{10}$; or (i) $n > 10^{11}$; (j) $n > 10^{12}$; (k) $n > 10^{13}$; (l) $n > 10^{14}$; or (m) $n > 10^{15}$. The library microcompartments may be selected from microdroplets, microparticles and microvesicles. Microparticulate library microcompartments, for example in the form of beads, are preferred.

The tagged chemical structures may be present in the library microcompartment(s) at a concentration sufficiently high as to permit cell-based or phenotypic screens, particularly homogeneous cell-based phenotypic assays. In certain embodiments, the tagged chemical structures may be present in the library microcompartment(s) at a concentration of at least: 0.1 nM, 0.5 nM, 1.0 nM 5.0 nM, 10.0 nM, 15.0 nM, 20.0 nM, 30.0 nM, 50.0 nM, 75.0 nM, 0.1 μM, 0.5 μM, 1.0 μM, 5.0 μM, 10.0 μM, 15.0 μM, 20.0 μM, 30.0 μM, 50.0 μM, 75.0 μM, 100.0 μM, 200.0 μM, 300.0 μM, 500.0 μM, 1 mM, 2 mM, 5 mM or 10 mM.

In other embodiments, the tagged chemical structures may be present in the library microcompartment(s) at a concentration of at least: 0.1 pM, 0.5 pM, 1.0 pM 5.0 pM, 10.0 pM, 15.0 pM, 20.0 pM, 30.0 pM, 50.0 pM, 75.0 pM 0.1 nM, 0.5 nM, 1.0 nM 5.0 nM, 10.0 nM, 15.0 nM, 20.0 nM, 30.0 nM, 50.0 nM, 75.0 nM, 0.1 μM, 0.5 μM, 1.0 μM, 5.0 μM, 10.0 μM, 15.0 μM, 20.0 μM, 30.0 μM, 50.0 μM, 75.0 μM, 100.0 μM, 200.0 μM, 300.0 μM, 500.0 μM, 1 mM, 2 mM, 5 mM or 10 mM.

In other embodiments, the tagged chemical structures may be present in the library microcompartments(s) at a concentration of: less than 1 μM; 1-100 μM; greater than 100 μM; 5-50 μM or 10-20 μM.

The spatial association of step (d) can be maintained by any suitable means, but preferred is micro-compartmentalization such that the TCS and its tag are confined in spatial proximity. Physical confinement can be achieved through the use of various micro-compartments, including microdroplets, microparticles, microwells, microarrays and microvesicles (as described in more detail below).

In preferred embodiments, the tags of step (d) are functionally or physically partitioned from the assay system. This prevents interference of the tags with the assay reagents. In such embodiments, the tags may be partitioned, sequestered, confined or located in or on a library microcompartment as defined above.

*Screening microcompartments*

In preferred embodiments, each of the library microcompartments may be placed into discrete screening microcompartments prior to step (c), for example by microencapsulation, picoinjection or microdroplet fusion. The screening microcompartments may take any form, but preferred are forms suitable for high throughput screening. Particularly preferred are screening microcompartments selected from microdroplets, microwells and/or microfluidic channels.

The screening microcompartments preferably contain the library microcompartments together with: (i) an aqueous solvent; and/or (iii) a gelling system, for example a hydrogel; and/or (iii) the assay system.

The screening microcompartments may conveniently comprise a gelling system which can be gelled prior to the identifying step (e) such that the library microcompartment becomes fixed within the gelled screening microcompartment. In such embodiments, the gelling step may be followed by a washing step prior to the identifying step (e), thereby removing potentially interfering reactants/products that might otherwise compromise the reliability of the decoding step.

In step (c) of the methods of the invention, the TCSs may be released into the screening microcompartments while the tags are: (a) retained in or on the library microcompartment, e.g. by covalent and/or hydrogen bonds; (b) sequestered by a functionalized surfactant at the surface of a library or screening microdroplet; or (c) sequestered by a component of the assay system.

The methods of the invention involve the step of releasing each chemical structure from its tag to produce a plurality of free, tagless chemical structures (TCSs). The TCSs are conveniently released into the screening microcompartments by diffusion, for example by diffusion directly from the library microcompartment.

The screening step (d) may be carried out within a screening microdroplet containing the assay system, the clonal population of TCSs and the library microcompartment containing the tags.

*Templated synthesis*

In certain embodiments, the encoding nucleic acid tag serves as a template for the chemical structure. In such embodiments, the library of tagged chemical structures is provided by nucleic acid-templated, for example DNA-templated, synthesis of the chemical structures. Any suitable templating technology may be employed, and suitable techniques are described, for example, in Mannocci et al. (2011) Chem. Commun., 47: 12747-12753; Kleiner et al. (2011) Chem Soc Rev. 40(12): 5707-5717 and Mullard (2016) Nature 530: 367-369. Also suitable is the DNA-routing approach developed by Professor Pehr Harbury and co-workers (Stanford University, USA). The DNA template may be patterned/configured in any way: for example the YoctoReactor system employs three-way DNA-hairpin-looped junctions to assist the library synthesis by transferring appropriate donor chemical moieties onto a core acceptor site (see WO2006/048025).

Alternative structural geometries are also available, such as 4-way DNA Holliday Junctions and hexagonal structures as described by Lundberg et al. (2008) Nucleic acids symposium (52): 683-684 and complex shapes and patterns created by the "scaffolded DNA origami" techniques reviewed by Rothemund (2006) Nature 440: 297-302.

*Reactor microcompartments*

In embodiments where the encoding nucleic acid tag serves as a template for the chemical structure, the templated synthesis may be preceded by a step comprising amplifying the encoding nucleic acid template, optionally by PCR. This amplification step may be carried out in a reactor microcompartment.

Thus, the invention contemplated library synthesis comprising the steps of: (a') providing a reactor microcompartment containing: (i) a clonal population of encoding templates; and (ii) a plurality of chemical substructures; and then (b') contacting the templates with the substructures within the reactor microcompartment under conditions whereby the substructures react to form a clonal population of chemical structures by nucleic acid-templated synthesis, thereby producing a reactor microcompartment containing a clonal population of chemical structures hybridized to encoding templates.

The reactor microcompartment in such embodiments may be selected from microwells, microarrays, microfluidic channels, microparticles, microvesicles and microdroplets. Preferred are microdroplets. The microcompartment may comprise a hydrogel and examples of such hydrogel-containing microcompartments are described herein.

Here, the method may further comprise the step of amplifying the number of chemical structures by: (i) dehybridizing the chemical structures from the encoding templates, and then (ii) contacting the dehybridized templates with unreacted substructures within the reactor microcompartment under conditions whereby the substructures react to form a further clonal population of chemical structures by nucleic acid-templated synthesis; and then (iii) repeating steps (i) and (ii).

In such embodiments, steps (i) and (ii) may be repeated until the chemical structures are present in the reactor microcompartment at a concentration of at least 0.1 nM, 0.5 nM, 1.0 nM 5.0 nM, 10.0 nM, 15.0 nM, 20.0 nM, 30.0 nM, 50.0 nM, 75.0 nM, 0.1 μM, 0.5 μM, 1.0 μM, 5.0 μM, 10.0 μM, 15.0 μM, 20.0 μM, 30.0 μM, 50.0 μM, 75.0 μM, 100.0 μM, 200.0 μM, 300.0 μM, 500.0 μM, 1 mM, 2 mM, 5 mM or 10 mM. Thus, steps (i) and (ii) may be repeated until the chemical structures are present in the reactor microcompartment at a concentration of 1-100 μM, 5-50 μM or 10-20 μM.

Templated synthesis in the context of the methods of the invention may comprise hybridization between nucleic acid coupled to the chemical structure and the nucleic acid of the encoding tag template.

*Clonal tagging of existing chemical libraries*

Templated synthesis is not an essential requirement for the methods of the invention, however, and the library of tagged chemical structures may be provided by any suitable means. For example, the library for use according to the invention may comprise a clonal population of chemical structures, and step (a) comprises the step of releasably linking an encoding tag to each of the chemical structures within said clonal population. The clonal population of chemical structures in such embodiments may be an element of a commercially available chemical library.

In such embodiments, the encoding tag may comprise a nucleic acid sequence, for example a DNA sequence. The tag may be releasably linked to the chemical structures at a plurality of different cross-linking sites, thereby mitigating any deleterious effects arising from crosslinking at particular site(s) on the chemical structure. In such embodiments, the encoding tags may be functionalized with a plurality of different cross-linking groups, so that tagging can occur at a plurality of distinct crosslinking sites.

Suitable nucleic acid-based tags are available commercially (e.g. from Twist Bioscience Corporation), but they

may be synthesised as described in e.g. WO2015/021080.

*Target cells*

The invention finds particular application in phenotypic, cell-based assays.

Thus, the assay system may be a homogeneous aqueous phase assay system, and the screening step (d) may comprise a phenotypic screen. In such embodiments, the assay system may comprise a live target cell. Any cell may be used in such embodiments, including prokaryotic and eukaryotic cells. Suitable prokaryotic cells include archaeal cells, for example selected from the phyla: (a) Crenarchaeota; (b) Euryarchaeota; (c) Korarchaeota; (d) Nanoarchaeota and (e) Thaumarchaeota, for example Haloferax volcanii and Sulfolobus spp..

Other prokaryotic cells suitable for use as target cells according to the invention include bacterial cells. In such embodiments, the target cell may be a pathogenic bacterium. Other bacterial target cells include cells selected from Gram-positive bacteria (for example, selected from *Enterococcus faecalis, Enterococcus faecium* and *Staphylococcus aureus);* Gram-negative bacteria (for example, selected from *Klebsiella pneumoniae, Acinetobacter baumanii, Escherichia coli, E. coli* ST131 strains, *Pseudomonas aeruginosa, Enterobacter cloacae, Enterobacter aerogenes* and *Neisseria gonorrhoeae*) and bacteria exhibiting an indeterminate Gram reaction.

Eukaryotic cells suitable for use as target cells according to the invention include: (a) fungal; (b) mammalian; (c) a higher plant cell; (d) protozoal; (e) a helminth cell; (f) algal; (g) a cell derived from a clinical tissue sample, for example a human patient sample and (h) an invertebrate cell.

Suitable mammalian cells include cancer cells, for example human cancer cells, muscle cells, human neuronal cells and others cells derived from a living human patient that show a disease relevant phenotype.

*Target proteins*

The assay system may comprise an isolated target protein or isolated target protein complex. For example, the target protein/protein complex may be an intracellular target protein/protein complex. The target protein/protein complex may be in solution, or may be comprised a membrane or transmembrane protein/protein complex. In such embodiments, the chemical structures may be screened for ligands which bind to the target protein/protein complex. The ligands may be inhibitors of the target protein/protein complex.

The assay system may comprise, or generate, a detectable label. In such embodiments, the detectable label may be linked to a target cell or to an isolated target protein or isolated target protein complex as described above.

The screening step may comprise FADS and/or FACS. The screening step may also comprise fluorescence analysis, including but not limited to FRET, FliM, fluorophore tagged antibody, fluorophore tagged DNA sequence or fluorescent dyes.

*Sequence-based structure-activity analysis*

The invention comprises the step of identifying a screened TCS by decoding a tag that is spatially associated therewith. In cases where the tag comprises a nucleic acid sequence, the decoding step may comprise sequencing the nucleic acid. In such embodiments, the method may further comprise comparing the sequences of a plurality of different screened TCSs. Such a step may be followed by a step of performing sequence activity relationship analysis on the screened TCSs, which can permit classification of screened library members into different chemotypes.

*Libraries*

In another aspect, the invention provides for use of an encoded chemical library in the method of the invention, which library comprises a number *n* of clonal populations of chemical structures each releasably linked to an encoding tag, each clonal population being confined to *n* discrete library microcompartments.

Here, the chemical structures may be linked to the encoding tags as described above, for example by a cleavable linker and/or by nucleic acid hybridization.

In another aspect, the invention provides for use of an encoded chemical library in the method of the invention, which library comprises a number *n* of clonal populations of free chemical structures, each clonal population being confined to *n* discrete library microcompartments, wherein the chemical structures are contained within the microcompartments together with encoding tags but are not covalently linked to the encoding tags.

In another aspect, the invention provides an assay composition for use in the method of the invention comprising the library of the invention in which the chemical structures contained within the microcompartments are in contact with an assay system. Here, the encoding tags may be functionally or physically partitioned from the assay system. For example, the chemical structures may be contained within a library microdroplet containing the assay system and the

encoding tags may be located in or on a microdroplet, bead or microvesicle encapsulated within said library microdroplet.

In another aspect, the invention provides a nucleic encoded chemical library reactor for use in the method of the invention comprising a microcompartment containing: (a) a clonal population of encoding nucleic acid template molecules; and (b) a plurality of chemical substructures, wherein the substructures are adapted for nucleic acid-templated assembly to form encoding nucleic-acid-tagged chemical structures in which the encoding tags are releasably linked to the chemical structures.

Other aspects and preferred embodiments of the invention are defined and described in the other claims set out below.

## Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

The term *Gram-positive bacterium* is a term of art defining a particular class of bacteria that are grouped together on the basis of certain cell wall staining characteristics.

The term *low G+C Gram-positive bacterium* is a term of art defining a particular subclass class of evolutionarily related bacteria within the Gram-positives on the basis of the composition of the bases in the DNA. The subclass includes *Streptococcus* spp., *Staphylococcus* spp., *Listeria* spp., *Bacillus* spp., *Clostridium* spp., *Enterococcus* spp. and *Lactobacillus* spp.).

The term *high G+C Gram-positive bacterium* is a term of art defining a particular subclass class of evolutionarily related bacteria within the Gram-positives on the basis of the composition of the bases in the DNA. The subclass includes actinomycetes (actinobacteria) including *Actinomyces* spp., *Arthrobacterspp., Corynebacterium* spp., *Frankia* spp., *Micrococcus* spp., *Micromonospora* spp., *Mycobacterium* spp., *Nocardia* spp., *Propionibacterium* spp. and *Streptomyces* spp.

The term *Gram-negative bacterium* is a term of art defining a particular class of bacteria that are grouped together on the basis of certain cell wall staining characteristics. Examples of Gram-negative bacterial genera include *Klebsiella, Acinetobacter, Escherichia, Pseudomonas, Enterobacter* and *Neisseria*.

As used herein, the term *assay system* defines means for detecting a desired activity. An assay system directly or indirectly produces a detectable and/or measurable signal when it contacts or reacts with a chemical structure present in the library and having the desired activity. The desired activity may be target protein binding, pharmacological activity, cell receptor binding, antibiotic, anticancer, antiviral, antifungal, antiparasitic, pesticide, pharmacological, immunological activity, production of any desired compound, increased production of a compound or breakdown of a specific product. The desired activity may be activity against a pharmacological target cell, cell protein or metabolic pathway. The desired activity may also be the ability to modulate gene expression, for example by decreasing or increasing the expression of one or more gene(s) and/or their temporal or spatial (e.g. tissue-specific) expression patterns. The desired activity may be binding activity, for example to act as a ligand to a target protein. The desired activity may also be one which is useful in various industrial processes, including bioremediation, microbially-enhanced oil recovery, sewage treatment, food production, biofuel production, energy generation, bio-production, bio-digestion/biodegradation, vaccine production and probiotic production. It could also be a chemical agent, such as a fluorophore, or pigment, specific chemical reaction or any chemical reaction that can be tied to a colour, matrix structure or refractive index change.

The assay system may comprise chemical indicators, including reporter molecules and detectable labels (as herein defined). It may be, for example, colorimetric (i.e. result in a coloured reaction product that absorbs light in the visible range), fluorescent (e.g. based on an enzyme converts a substrate to a reaction product that fluoresces when excited by light of a particular wavelength) and/or luminescent (e.g. based on bioluminescence, chemiluminescence and/or photoluminescence).

The assay system may comprise cells, for example the target cells as described herein. The assay system may also comprise proteins, for example the target proteins as described herein. Alternatively, or in addition, the assay system may comprise cell fractions, cellular components, tissue, tissue extracts, multi-protein complexes, membrane-bound proteins membrane fractions and/or organoids.

The term *ligand* as used herein to define a binding partner for a biological target molecule *in vivo* (for example, an enzyme or receptor). Such ligands therefore include those which bind (or directly physically interact) with the target *in vivo* irrespective of the physiological consequences of that binding. Thus, the ligands of the invention may bind the target as part of a cellular signalling cascade in which the target forms a part. Alternatively, they may bind the target in the context of some other aspect of cellular physiology. In the latter case, the ligands may for example bind the target at the cell surface without triggering a signalling cascade, in which case the binding may affect other aspects of cell function. Thus, the ligands of the invention may bind the target at the cell surface and/or intracellularly.

As used herein, the term *small molecule* means any molecule having a molecular weight of 1000 Da or less, for example less than 900 Da, less than 800 Da, less than 600 Da or less than 500 Da. Preferably, the chemical structures present in the libraries of the invention may be small molecules as herein defined, particularly small molecules having a molecular weight of less than 600 Da.

As used herein, the term *large molecule* means any molecule having a molecular weight greater than 1000 Da.

As used herein, the term *antibody* defines whole antibodies (including polyclonal antibodies and monoclonal antibodies (mAbs)). The term is also used herein to refer to antibody fragments, including F(ab), F(ab'), F(ab')2, Fv, Fc3 and single chain antibodies (and combinations thereof), which may be produced by recombinant DNA techniques or by enzymatic or chemical cleavage of intact antibodies. The term "antibody" is also used herein to cover bispecific or bifunctional antibodies which are synthetic hybrid antibodies having two different heavy/light chain pairs and two different binding sites. Bispecific antibodies can be produced by a variety of methods including fusion of hybridomas or linking of Fab' fragments. Also covered by the term "antibody" are chimaeric antibodies (antibodies having a human constant antibody immunoglobulin domain coupled to one or more non-human variable antibody immunoglobulin domain, or fragments thereof). Such chimaeric antibodies therefore include "humanized" antibodies. Also covered by the term "antibody" are minibodies (see WO 94/09817), single chain Fv-Fc fusions and human antibodies produced by transgenic animals The term "antibody" also includes multimeric antibodies and higher-order complexes of proteins (e.g. heterodimeric antibodies).

As used herein, the terms *peptide, polypeptide* and *protein* are used interchangeably to define organic compounds comprising two or more amino acids covalently joined by peptide bonds. The corresponding adjectival term "peptidic" is to be interpreted accordingly. Peptides may be referred to with respect to the number of constituent amino acids, i.e., a dipeptide contains two amino acid residues, a tripeptide contains three, etc. Peptides containing ten or fewer amino acids may be referred to as oligopeptides, while those with more than ten amino acid residues are polypeptides. Such peptides may also include any of the modifications and additional amino and carboxy groups.

As used herein, the term *click chemistry* is a term of art introduced by Sharpless in 2001 to describe reactions that are high yielding, wide in scope, create only by-products that can be removed without chromatography, are stereospecific, simple to perform and can be conducted in easily removable or benign solvents. It has since been implemented in many different forms, with wide applications in both chemistry and biology. A subclass of click reactions involve reactants which are inert to the surrounding biological milieu. Such click reactions are termed *bioorthogonal.* Bioorthogonal reactant pairs suitable for bioorthogonal click chemistry are molecular groups with the following properties: (1) they are mutually reactive but do not significantly cross-react or interact with cellular biochemical systems in the intracellular milieu; (2) they and their products and by-products are stable and nontoxic in physiological settings; and (3) their reaction is highly specific and fast. The reactive moieties (or click reactants) may be selected by reference to the particular click chemistry employed, and so any of a wide range of compatible pairs of bioorthogonal click reactants known to those skilled in the art may be used according to the invention, including Inverse electron demand Diels-Alder cycloaddition reaction (IEDDA), Strain-promoted alkyne azide cycloaddition (SPAAC) and Staudinger ligation.

The term *isolated* is used herein in relation to any material (e.g. a chemical compound, assay reagent, target protein or target cell) to indicate that the material exists in a physical *milieu* distinct from that in which it occurs in nature. For example, isolated cells may be substantially isolated (for example purified) with respect to the complex tissue *milieu* in which they naturally occur. Isolated cells may, for example, be purified or separated. In such cases, the isolated cells may constitute at least 60%, 70%, 75%, 80%, 85%, 90%, 95%, 96%, 97%, 98%, 99%, or 100% of the total cell types present. Isolated cells may be obtained by routine techniques known to those skilled in the art, including FACS, density gradient centrifugation, enrichment culture, selective culture, cell sorting and panning techniques using immobilized antibodies against surface proteins.

When the isolated material is purified, the absolute level of purity is not critical and those skilled in the art can readily determine appropriate levels of purity according to the use to which the material is to be put. Preferred, however, are purity levels of at least 60%, 70%, 75%, 80%, 85%, 90%, 95%, 96%, 97%, 98%, 99%, or 100% w/w. In some circumstances, the isolated material forms part of a composition (for example a more or less crude cellular extract containing many other cellular components) or buffer system, which may contain other components.

The term *contacting* is used herein refers to the process of allowing at least two distinct moieties or systems (e.g. chemical structures and an assay system) to become sufficiently proximal to react, interact or physically touch.

The term *detectable label* is used herein to define a moiety detectable by spectroscopic, fluorescent, photochemical, biochemical, immunochemical, chemical, electrochemical, radiofrequency or by any other physical means. Suitable labels include fluorescent proteins, fluorescent dyes, electron-dense reagents, enzymes (e.g., as commonly used in an ELISA), biotin, digoxigenin, or haptens and proteins or other entities which can be made detectable, for example by incorporating a radio- or fluorescent label into a peptide or antibody specifically reactive with a target peptide.

As used herein, the term *microdroplet* defines a small, discrete volume of a fluid, liquid or gel having a diameter of 0.1 μm to 1000 μm and/or a volume of between 5 x $10^{-7}$ pL and 500 nL. Typically, the microdroplets have a diameter of less than 1000 μm, for example less than 500 μm, less than 500 μm, less than 400 μm, less than 300 μm, less than 200 μm, less than 100 μm, less than 50 μm, less than 40 μm, less than 30 μm, less than 20 μm, less than 10 μm, less than 5 μm, or less than 1 μm. Thus, the microdroplets may be substantially spherical with a diameter of: (a) less than 1 μm; (b) less than 10 μm; (c) 0.1-10 μm; (d) 10 μm to 500 μm; (b) 10 μm to 200 μm; (c) 10 μm to 150 μm; (d) 10 μm to 100 μm; (e) 10 μm to 50 μm; or (f) about 100 μm.

The microdroplets of the invention are typically comprised of an isolated portion of a first fluid, liquid or gel that is completely surrounded by a second fluid, liquid or gel (e.g. an immiscible liquid or a gas). In some cases, the droplets may be spherical or substantially spherical. However, in some cases, the microdroplets may be non-spherical and have an irregular shape (for example due to forces imposed by the external environment or during physical manipulation during the assay and screening processes described herein). Thus, microdroplets may be substantially cylindrical, plug-like and or oval in shape (for example in circumstances where they conform to the geometry of a surrounding microchannel).

As used herein, the term *microparticle* defines a particle having a diameter of less than 1000 μm, for example less than 500 μm, less than 500 μm, less than 400 μm, less than 300 μm, less than 200 μm, less than 100 μm, less than 50 μm, less than 40 μm, less than 30 μm, less than 20 μm, less than 10 μm, less than 5 μm, or less than 1 μm. The microparticles are preferably non-planar and may have a largest dimension of (or be substantially spherical with a diameter of): (a) 10 μm to 500 μm; (b) 10 μm to 200 μm; (c) 10 μm to 150 μm; (d) 10 μm to 100 μm; (e) 10 μm to 50 μm; or (f) about 100 μm. Microparticles may therefore be encapsulated within microdroplets as herein defined. Microparticles may be formed from rigid solids, flexible gels, porous solids, porous gels or networks or matrices of rigid or semi-rigid fibrils or tubules.

As used herein, the term *bead* is used to define a solid microparticle. Preferred beads are formed from gels (including hydrogels such as agarose), for example by fragmentation of a gelled bulk composition or moulding from a pre-gelled state. The beads may be functionalized with reactive groups or moieties, such as streptavidin, amine or cyanogen bromide. Alternatively, these beads could be a solid support such as those made of silicon, polystyrene (PS), crosslinked poly(styrene/divinylbenzene) (P[S/DVB]), and poly(methyl methacrylate) (PMMA).

The term *microvesicle* is used herein to define a hollow microparticle comprising an outer wall or membrane enclosing an internal volume, such as a liposome.

The microdroplets, microparticles and microvesicles of the invention may be monodisperse. The term *monodisperse,* as applied to the microdroplets and microparticles for use according to the invention, defines a microdroplet/microparticle population having a coefficient of droplet/particle size dispersion, ε, of not more than 1.0, not more than 0.5, and preferably not more than 0.3. Said coefficient ε is defined by the following equation:

$$\varepsilon=({}^{90}D_p - {}^{10}D_p)/{}^{50}D_p \quad (1)$$

where ${}^{10}D_p$, ${}^{50}D_p$ and ${}^{90}D_p$ are the particle sizes when the cumulative frequencies estimated from a relative cumulative particle size distribution curve for the emulsion are 10%, 50% and 90%, respectively. The case where ε=0 means an ideal state in which emulsion particles show no particle size scattering at all.

As used herein, the term *encoding tag* is used, in relation to a chemical structure, to define a moiety or agent which contains information which uniquely identifies the chemical structure or its reaction history, thereby serving as a unique identifier of that particular chemical structure (i.e. the tag "encodes" that structure and serves as a molecular "barcode"). The information may be encoded in any form, but in preferred embodiments the tag is a nucleic acid (for example DNA) tag in which the information is encoded in the sequence of the nucleic acid. However, other tags may be used, including non-DNA tags, non-RNA tags, modified nucleic acid tags, peptide tags, light-based barcodes (e.g. quantum dots) and RFID tags.

As used herein, the term *clonal* in relation to chemical structures defines a population of chemical structures each of which is encoded by a common tag. The chemical structures may be chemically identical, or may differ only in respect to the nature and/or position of the cleavable linker coupling the chemical structure to its cognate encoding tag (or in respect to a scar remaining after cleavage of such a linker).

As used herein, the term *free* as applied to chemical structures is used to define a chemical structure which is not bound to a solid phase. In some embodiments, the term defines a chemical structure which is not covalently bound

to a solid phase. Free chemical structures may therefore enter a liquid phase and/or enter solution, and may in some embodiments may be bound and/or taken up by cells present in the assay system.

As used herein, the term *microwell* (used herein interchangeably with the term *well*) refers to a chamber having a volume of less than 1ml. Microwells having dimensions in the nm range can be fabricated by electron beam lithography (see e.g. Odom et al. (2002) J. AM. CHEM. SOC. 124: 12112-12113). Microwells are typically disposed on a solid substrate or microtiter plate (also referred to as a microplate, microwell or multiwall plate), this typically taking the form of a flat plate with multiple "wells" used as small test tubes. Microplates typically have 6, 24, 96, 384 or 1536 sample microwells arranged in a 2:3 rectangular matrix.

As used herein, the term *self-immolative linker* defines a linker comprising a self-immolative chemical group (which may be referred to herein as a self-immolative moiety or "SIM") capable of directly or indirectly (e.g. *via* a peptide moiety) covalently linking the chemical structure and its encoding tag to form a stable tagged chemical structure, and which is capable of releasing the encoding tag from the chemical structure by a mechanism involving spontaneous release of the chemical structure (for example *via* an electronic cascade triggered by enzymatic cleavage that leads to the expulsion of a leaving group and release of the free chemical structure).

Micro-compartments

The invention involves the maintenance of a spatial association between a free chemical structure and its encoding tag. This is conveniently achieved by micro-compartmentalization, which is a process of physically confining the chemical structure and its tag such that they are maintained in physical proximity (i.e. each chemical structure is located within 1000μm (for example, within 500μm, 250μm, 100μm, 50μm, 25μm, 10μm or 1μm) of its corresponding encoding tag). Physical confinement can be achieved through the use of various micro-compartments, including microdroplets, microparticles, microwells and microvesicles, as explained below. Physical confinement permits the identification of the chemical structure of screening hits by decoding the spatially-associated tag.

Microdroplets

Suitable materials and methods for preparing and processing microdroplets suitable for use in micro-compartmentalization according to the invention form part of the common general knowledge of those skilled in the art, being described, for example, in WO2010/009365, WO2006/040551, WO2006/040554, WO2004/002627, WO2004/091763, WO2005/021151, WO2006/096571, WO2007/089541, WO2007/081385 and WO2008/063227.

The size of the microdroplets will be selected by reference to the nature of the chemical structures and assay system to be encapsulated. The microdroplets may be substantially spherical with a diameter of: (a) less than 1 μm; (b) less than 10 μm; (c) 0.1-10 μm; (d) 10 μm to 500 μm; (b) 10 μm to 200 μm; (c) 10 μm to 150 μm; (d) 10 μm to 100 μm; (e) 10 μm to 50 μm; or (f) about 100 μm.

The microdroplets are preferably uniform in size such that the diameter of any droplet within the library will vary less than 5%, 4%, 3%, 2%, 1% or 0.5% when compared to the diameter of other droplets within the same library. In some embodiments, the microdroplets are monodisperse. However, polydisperse microdroplets may also be used according to the invention.

In single W/O type emulsions, the carrier liquid may be any water-immiscible liquid, for example an oil, optionally selected from: (a) a hydrocarbon oil; (b) a fluorocarbon oil; (c) an ester oil; (d) a silicone oil; (e) an oil having low solubility for biological components of the aqueous phase; (f) an oil which inhibits molecular diffusion between microdroplets; (g) an oil which is hydrophobic and lipophobic; (h) an oil having good solubility for gases; and/or (i) combinations of any two or more of the foregoing.

Thus, the microdroplets may be comprised in a W/O emulsion wherein the microdroplets constitute an aqueous, dispersed, phase and the carrier liquid constitutes a continuous oil phase.

In other embodiments, the microdroplets are comprised in a W/O/W double emulsion and the carrier liquid may an aqueous liquid. In such embodiments, the aqueous liquid may be phosphate buffered saline (PBS).

The microdroplets may therefore be comprised in a W/O/W double emulsion wherein the microdroplets comprise: (a) an inner core of aqueous growth media enveloped in an outer oil shell as the dispersed phase, and (b) the carrier liquid as the continuous aqueous phase. It will of course be appreciated that O/W/O droplets may be particularly useful for screening non-biological entities.

Surfactants

In embodiments where the microdroplets are comprised in an emulsion, the carrier liquid may constitute the continuous phase and the microdroplets the dispersed phase, and in such embodiments the emulsion may further comprise a surfactant and optionally a co-surfactant.

The surfactant and/or co-surfactant may be located at the interface of the dispersed and continuous phases, and when the microdroplets are comprised in a W/O/W double emulsion the surfactant and/or co-surfactant may be located at the interface of aqueous core and oil shell and at the interface of the oil shell and outer continuous phase.

A wide range of suitable surfactants are available, and those skilled in the art will be able to select an appropriate surfactant (and co-surfactant, if necessary) according to the selected screening parameters. For example, suitable surfactants are described in Bernath et al. (2004) Analytical Biochemistry 325: 151-157; Holtze and Weitz (2008) Lab Chip 8(10): 1632-1639; and Holtze et al. (2008) Lab Chip. 8(10):1632-1639. Other suitable surfactants, including fluorosurfactants in particular, are described in WO2010/009365 and WO2008/021123.

The surfactant(s) and/or co-surfactant(s) are preferably incorporated into the W/O interface(s), so that in embodiments where single W/O type emulsions are used the surfactant(s) and or co-surfactant(s) may be present in at the interface of the aqueous growth medium microdroplets and the continuous (e.g., oil) phase. Similarly, where double W/O/W type emulsions are used for co-encapsulation according to the invention, the surfactant(s) and or co-surfactant(s) may be present at either or both of the interfaces of the aqueous core and the immiscible (e.g. oil) shell and the interface between the oil shell and the continuous aqueous phase.

The surfactant(s) are preferably biocompatible. For example, the surfactant(s) may be selected to be non-toxic to any cells used in the screen. The selected surfactant(s) may also have good solubility for gases, which may be necessary for the growth and/or viability of any encapsulated cells.

Biocompatibility may be determined by any suitable assay, including assays based on tests for compatibility with a reference sensitive biochemical assay (such as *in vitro* translation) which serves as a surrogate for biocompatibility at the cellular level. For example, *in vitro* translation (IVT) of plasmid DNA encoding the enzyme β-galactosidase with a fluorogenic substrate (fluorescein di- β -D-galactopyranoside (FDG)) may be used as an indicator of biocompatibility since a fluorescent product is formed when the encapsulated DNA, the molecules involved in transcription and translation, and the translated protein do not adsorb to the drop interface and the higher-order structure of the protein remains intact.

Biocompatibility may also be determined by growing cells to be used in an assay in the presence of the surfactant and staining the cell with antibodies or viable cell dyes and determining the overall viability for the cell population compared to a control in the absence of the surfactant.

The surfactant(s) may also prevent the adsorption of biomolecules at the microdroplet interface. The surfactant may also function to *isolate* the individual microdroplets (and the corresponding microcultures). The surfactant preferably stabilizes (i.e. prevents coalescence) of the microdroplets. Stabilization performance can be monitored by e.g. phase-contrast microscopy, light scattering, focused beam reflectance measurement, centrifugation and/or rheology.

The surfactant may also form a functional part of the assay system, and may for example act to partition or sequester reactants and/or analytes and/or other moieties present in the assay (such as released tags) from other components. For example, a nickel complex in a hydrophilic head group of a functional surfactant can concentrate histidine-tagged proteins at the surface (see e.g. Kreutz et al. (2009) J Am Chem Soc. 131(17): 6042-6043). Such functionalized surfactants may also act as catalysts for small molecule synthesis (see e.g. Theberge et al. (2009) Chem. Commun.: 6225-6227). They may also be used to cause cell lysis (see e.g. Clausell-Tormos et al. (2008) Chem Biol. 15(5): 427-37). The present invention therefore contemplates the use of such functionalized surfactants.

*Oils for use in emulsion co-encapsulation*

While it will be appreciated than any liquid immiscible with the discontinuous phase may be used in the formation of microdroplet emulsions for use according to the invention, the immiscible fluid is typically an oil.

Preferably, an oil is selected having low solubility for biological components of the aqueous phase. Other preferred functional properties include tunable (e.g. high or low) solubility for gases, the ability to inhibit molecular diffusion between microdroplets and/or combined hydrophobicity and lipophobicity. The oil may be a hydrocarbon oil, for example light mineral oils, fluorocarbon oils, silicone oils or ester oils. Mixtures of two or more of the above-described oils are also preferred.

Examples of suitable oils are described in WO2010/009365, WO2006/040551, WO2006/040554, WO2004/002627, WO2004/091763, WO2005/021151, WO2006/096571, WO2007/089541, WO2007/081385 and WO2008/063227.

Processes for microdroplet emulsification

A wide range of different emulsification methods are known to those skilled in the art, any of which may be used to create the microdroplets of the invention.

Many emulsification techniques involve mixing two liquids in bulk processes, often using turbulence to enhance drop breakup. Such methods include vortexing, sonication, homogenization or combinations thereof.

In these "top-down" approaches to emulsification, little control over the formation of individual droplets is available, and a broad distribution of microdroplet sizes is typically produced. Alternative "bottom up" approaches operate at the level of individual drops, and may involve the use of microfluidic devices. For example, emulsions can be formed in a microfluidic device by colliding an oil stream and a water stream at a T-shaped junction: the resulting microdroplets vary in size depending on the flow rate in each stream.

A preferred process for producing microdroplets for use according to the invention comprises flow focusing (as described in e.g. Anna et al. (2003) Appl. Phys. Lett. 82(3): 364-366). Here, a continuous phase fluid (focusing or sheath fluid) flanking or surrounding the dispersed phase (focused or core fluid), produces droplet break-off in the vicinity of an orifice through which both fluids are extruded. A flow focusing device consists of a pressure chamber pressurized with a continuous focusing fluid supply. Inside, one or more focused fluids are injected through a capillary feed tube whose extremity opens up in front of a small orifice linking the pressure chamber with the external ambient environment. The focusing fluid stream moulds the fluid meniscus into a cusp giving rise to a steady micro or nano-jet exiting the chamber through the orifice; the jet size is much smaller than the exit orifice. Capillary instability breaks up the steady jet into homogeneous droplets or bubbles.

The feed tube may be composed of two or more concentric needles and different immiscible liquids or gases be injected leading to compound drops. Flow focusing ensures an extremely fast as well as controlled production of up to millions of droplets per second as the jet breaks up.

Other microfluidic processing techniques include pico-injection, a technique in which reagents are injected into aqueous drops using an electric field (see e.g. Eastburn et al. (2013) Picoinjection Enables Digital Detection of RNA with Droplet RT-PCR. PLoS ONE 8(4): e62961. doi:10.1371/journal.pone.0062961). Microdroplets can be fused to bring two reagents together, for example actively by electrofusion (see e.g. Tan and Takeuchi (2006) Lab Chip. 6(6): 757-63) or passively (as reviewed by Simon and Lee (2012) "Microdroplet Technology", in Integrated Analytical Systems pp 23-50, 10.1007/978-1-4614-3265-4_2).

In all cases, the performance of the selected microdroplet forming process may be monitored by phase-contrast microscopy, light scattering, focused beam reflectance measurement, centrifugation and/or rheology.

Fluorescence-Activated Droplet Sorting

As explained herein, the methods of invention are suitable for high-throughput screening, since they involve compartmentalizing the screening assay in tiny volumes of medium in the form of discrete microdroplets. This permits each microdroplet to be treated as a separate culture vessel, permitting rapid screening of large numbers of individual liquid cocultures using established microfluidic and/or cell-sorting methodologies.

Thus, following the co-encapsulation step, the resultant microdroplets may be sorted by adapting well-established fluorescence-activated cell sorting (FACS) devices and protocols. This technique has been termed *Fluorescence-Activated Droplet Sorting* (FADS), and is described, for example, in Baret et al. (2009) Lab Chip 9: 1850-1858. Any change that can be detected by using a fluorescent moiety can be screened for.

The target cells may be fluorescently labelled to enable FADS. A variety of fluorescent proteins can be used as labels for this purpose, including for example the wild type green fluorescent protein (GFP) of *Aequorea victoria* (Chalfie et al. 1994, Science 263:802-805), and modified GFPs (Heim et al. 1995, Nature 373:663-4; PCT publication WO 96/23810). Alternatively, DNA2.0's IP-Free© synthetic non-Aequorea fluorescent proteins can be used as a source of different fluorescent protein coding sequences that can be amplified by PCR or easily excised using the flanking Bsal restriction sites and cloned into any other expression vector of choice.

Transcription and translation of this type of reporter gene leads to accumulation of the fluorescent protein in the cells, so rendering them amenable to FADS.

Alternatively, a huge range of dyes are available that fluoresce at specific levels and conditions within a cell. Examples include those available from Molecular probes (Thermo Scientific). Alternatively cellular components can be detected with antibodies and these can be stained with any number of fluorophores using commercially available kits. Similarly DNA sequences can be introduced labelled with fluorophores into a cell and will adhere by hybridisation to complementary DNA and RNA sequences in the cell allowing direct detection of gene expression in a process called Fluorescent in-situ hybridisation (FISH).

Any labelling process that can be applied in current high content screening can be applied to FADS and be detected as a change in fluorescent signal relative to controls.

Tag templated synthesis

The encoding tag may serve as a template which directs the synthesis of the encoded chemical structure by exploiting DNA templating techniques. Suitable techniques are known to those skilled in the art, and are described, for example, in Mannocci et al. (2011) Chem. Commun., 47: 12747-12753; Kleiner et al. (2011) Chem Soc Rev. 40(12): 5707-5717; and Mullard (2016) Nature 530: 367-369. Thus, any suitable templating technique may be employed, including

those based on the DNA-templated synthesis (DTS) as described by Professor David Liu and co-workers (Harvard University, USA) and later commercialized by Ensemble Discovery (Cambridge, MA, USA). Here, chemical reactions are promoted by

bringing DNA-linked reagents into proximity through Watson-Crick base pairing.

Also suitable is the DNA-routing approach developed by Professor Pehr Harbury and co-workers (Stanford University, USA), and the YoctoReactor system developed by Vipergen (Copenhagen, Denmark). In the latter approach, three-way DNA-hairpin-looped junctions assist the library synthesis by transferring appropriate donor chemical moieties onto a core acceptor site (see WO2006/048025. Alternative structural geometries are also available, such as 4-way DNA Holliday Junctions and hexagonal structures as described by Lundberg et al. (2008) Nucleic acids symposium (52): 683-684 and complex shapes and patterns created by the "scaffolded DNA origami" techniques reviewed by Rothemund (2006) Nature 440: 297-302.

Proximity-triggered reactions may be promoted, for example, by the use of click chemistry (as herein defined).

Tag sequencing

Any suitable sequencing technique can be used, including Sanger sequencing, but preferred are sequencing methods and platforms termed next-generation sequencing (NGS), also known as high-throughput sequencing. There are many commercially available NGS sequencing platforms that are suitable for use in the methods of the invention. Sequencing-by-synthesis (SBS)-based sequencing platforms are particularly suitable. The Illumina™ system (which generates millions of relatively short sequence reads (54, 75 or 100bp) is particularly preferred.

Other suitable techniques include methods based on reversible dye-terminators, including those sold by Illumina™. Here, DNA molecules are first attached to primers on a slide and amplified so that local clonal colonies are formed (bridge amplification). Four types of ddNTPs are added, and non-incorporated nucleotides are washed away. Unlike pyrosequencing, the DNA can only be extended one nucleotide at a time. A camera takes images of the fluorescently labeled nucleotides then the dye along with the terminal 3' blocker is chemically removed from the DNA, allowing a next cycle.

Other systems capable of short sequence reads include SOLiD™ and Ion Torrent technologies (both sold by Thermo Fisher Scientific Corporation). SOLiD™ technology employs sequencing by ligation. Here, a pool of all possible oligonucleotides of a fixed length are labeled according to the sequenced position. Oligonucleotides are annealed and ligated; the preferential ligation by DNA ligase for matching sequences results in a signal informative of the nucleotide at that position. Before sequencing, the DNA is amplified by emulsion PCR. The resulting bead, each containing only copies of the same DNA molecule, are deposited on a glass slide. The result is sequences of quantities and lengths comparable to Illumina sequencing.

Ion Torrent Systems Inc. have developed a system based on using standard sequencing chemistry, but with a novel, semiconductor-based detection system. This method of sequencing is based on the detection of hydrogen ions that are released during the polymerisation of DNA, as opposed to the optical methods used in other sequencing systems. A microwell containing a template DNA strand to be sequenced is flooded with a single type of nucleotide. If the introduced nucleotide is complementary to the leading template nucleotide it is incorporated into the growing complementary strand. This causes the release of a hydrogen ion that triggers a hypersensitive ion sensor, which indicates that a reaction has occurred. If homopolymer repeats are present in the template sequence multiple nucleotides will be incorporated in a single cycle. This leads to a corresponding number of released hydrogens and a proportionally higher electronic signal.

Methods such as or similar to that that used by Oxford Nanopore Technologies, where nucleic acid or other macromolecules are passed through a nano scale pore and the specific ionic current changes or electrical signals generated are used to identify it. For example, the individual bases of an oligonucleotide can be identified either as successive bases pass through the ion pore as part of the oligonucleotide or as single nucleotides after successive cleavage steps.

Cleavable linkers

Any cleavable linker may be used to link the chemical structure and its encoding tag, provided that: (a) the link can be broken to leave the coding information of the tag intact; and (b) the encoded chemical structure is released in a form in which it is completely or substantially free of linker residues, so that its activity in the screen is uncompromised by "scars" remaining after linker cleavage. It will be appreciated that some linker "scars" may be tolerated, such as -OH and/or -SH groups. The method of cleavage is preferably compatible with the assay system.

A wide range of suitable cleavable linkers are known to those skilled in the art, and suitable examples are described by Leriche et al. (2012) Bioorganic & Medicinal Chemistry 20 (2): 571-582. Suitable linkers therefore include enzymatically cleavable linkers; nucleophile/base-sensitive linkers; reduction sensitive linkers; photocleavable linkers; electrophile/acid-sensitive linkers; metal-assisted cleavage-sensitive linkers; oxidation-sensitive linkers; and combinations of two or more of the foregoing.

Enzyme cleavable linkers are described, for example, in: WO 2017/089894; WO 2016/146638; US2010273843; WO 2005/112919; WO 2017/089894; de Groot et al. (1999) J. Med. Chem. 42: 5277; de Groot et al. (2000) J Org. Chem. 43: 3093 (2000); de Groot et al., (2001) J Med. Chem. 66: 8815; WO 02/083180; Carl et al. (1981) J Med. Chem. Lett. 24: 479; Studer et al. (1992) Bioconjugate Chem. 3 (5): 424-429; Carl et al. (1981) J. Med. Chem. 24 (5): 479-480 and Dubowchik et al. (1998) Bioorg & Med. Chern. Lett. 8: 3347. They include linkers cleavable by enzymes selected from: β-glucuronidase, lysosomal enzymes, TEV, trypsin, thrombin, cathepsin B, B and K, caspase, matrix metalloproteinase sequences, phosphodiester, phospholipid, ester and β-galactose. Nucleophile/base cleavable linkers include: dialkyl dialkoxysilane, cyanoethyl group, sulfone, ethylene glycol disuccinate, 2-N-acryl nitrobenzenesulfonamide, α-thiophenylester, unsaturated vinyl sulphide, sulphonamide, malondialdehyde indole derivative, levulinoyl ester, hydrazine, acylhydrazone, alkyl thioester. Reduction cleavable linkers include disulphide bridges and azo compounds. Radiation cleavable linkers include: 2-Nitrobenzyl derivatives, phenacyl ester, 8-quinolinyl benzenesulphonate, coumarin, phosphotriester, bis-arylhydrazone, bimane bi-thiopropionic acid derivatives. Electrophilie/acid cleavable linkers include: paramethoxybenzyl derivatives, tert-butylcarbamate analogues, dialkyl or diaryl dialkoxysilane, orthoester, acetal, aconityl, hydrazine, β-thiopropionate, phosphoramadite, imine, trityl, vinyl ether, polyketal, alkyl 2-(diphenylphosphino) benzoate derivatives. Organometallic/metal catalysed cleavable linkers include: allyl esters, 8-hydroxylquinoline ester and picolinate ester. Linkers cleavable by oxidation include: vicinal diols and selenium compounds.

In certain embodiments, the cleavable linker comprises a combination of covalent and noncovalent bonds (for example hydrogen bonds arising from nucleic acid hybridization).

Cleavable (for example enzyme cleavable) peptide linkers may contain a peptide moiety that consists of single amino acid, or a dipeptide or tripeptide sequence of amino acids. The amino acids may be selected from natural and non-natural amino acids, and in each case the side chain carbon atom may be in either D or L (R or S) configuration. Exemplary amino acids include alanine, 2-amino-2-cyclohexylacetic acid, 2-amino-2-phenylacetic acid, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, γ-aminobutyric acid, β,β-dimethyl γ-aminobutyric acid, α,α-dimethyl γ-aminobutyric acid, ornithine, and citrulline (Cit). Suitable amino acids also include protected forms of the foregoing amino acids in which the reactive functionality of the side chains is protected. Such protected amino acids include lysine protected with acetyl, formyl, triphenylmethyl (trityl), and monomethoxytrityl (MMT). Other protected amino acid units include arginine protected tosyl or nitro groups and ornithine-protected with acetyl or formyl groups.

*Self-immolative linkers*

Particularly suitable for use as cleavable linkers in the invention are self-immolative linkers comprising: (a) a cleavage moiety; and (b) a self-immolative moiety ("SIM").

Such linkers may be used as shown diagrammatically in Figure 8, which shows spontaneous elimination of the SIM following cleavage to release the free chemical structure.

Particularly suitable are self-immolative linkers comprising: (a) an enzymatic cleavage moiety; and (b) a SIM. In such embodiments, the enzymatic cleavage moiety may be a peptide sequence (cleavable with proteases) or a non-peptide enzymatically cleavable group, for example a glucuronide moiety incorporating a hydrophilic sugar group cleavable by beta-glucuronidase (as explained in McCombs and Owen (2015) Antibody Drug Conjugates: Design and Selection of Linker, Payload and Conjugation Chemistry The AAPS Journal 17(2): 339-351) and shown below:

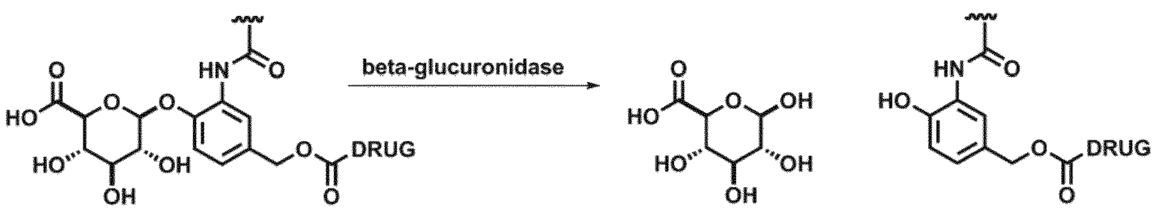


Suitable β-glucuronide-based linkers are described in WO 2007/011968, US 20170189542 and WO 2017/089894. Such linkers may therefore have the formula:

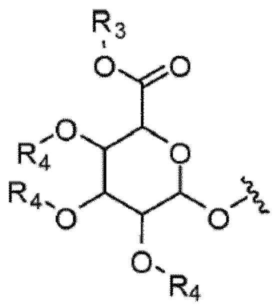

wherein $R_3$ is hydrogen or a carboxyl protecting group and each $R_4$ is independently hydrogen or a hydroxyl protecting group.

The SIM of the self-immolative linkers for use in the invention may be selected from a substituted alkyl, unsubstituted alkyl, substituted heteroalkyl, unsubstituted heteroalkyl, unsubstituted heterocycloalkyl, substituted heterocycloalkyl, substituted and unsubstituted aryl or substituted and unsubstituted heteroaryl. Suitable SIMs therefore include the p-aminobenzyl alcohol (PAB) unit and aromatic compounds that are electronically similar to the PAB group (such as the 2- aminoimidazol-5-methanol derivatives described by Hay et al. (1999) Bioorg. Med. Chem. Lett. 9: 2237) and ortho- or para-aminobenzylacetals.

Other suitable SIMs are those that undergo cyclization upon amide bond hydrolysis, for example the substituted and unsubstituted 4-aminobutyric acid amides described by Rodrigues et al. (1995) Chemistry Biology 2: 223). Yet further suitable SIMs include appropriately substituted bicyclo[2.2.1] and bicyclo[2.2.2] ring systems as described by Storm et al. (1972) J. Amer. Chem. Soc. 94: 5815 and various 2-aminophenylpropionic acid amides (see, e.g., Amsberry et al. (1990) J. Org. Chem. 55: 5867).

Particularly suitable are self-immolative peptide linkers comprising a peptide as cleavage moiety. Figures 9 and 10 show the construction and use of an encoded chemical library using such a linker. Here, the cleavable peptide is the dipeptide valine-citrulline and the SIM is p-aminobenzyl alcohol (PAB). In such embodiments, enzymatic cleavage of the amide-linked PAB triggers a 1,6-elimination of carbon dioxide and concomitant release of the free chemical structure. As also shown in Fig 9, the encoding tag and chemical structure(s) may be linked *via* a bead, and a single bead may be loaded with a plurality (where $n$ >1) of chemical structures, for example such that the ratio of encoding tag(s) to linked chemical structures is 1:10 to 1:1000. In such embodiments, the relatively small size of the peptide linker permits enhanced rates of diffusion and higher bead loadings, while the chemical structure needs only a single amine for functionalization.

Non-limiting examples of suitable cleavage moieties and SIMs for use as self-immolative linkers according to the invention are described, for example, in: WO 2017/089894; WO 2016/146638; US2010273843; WO 2005/112919; WO 2017/089894; de Groot et al. (1999) J. Med. Chem. 42: 5277; de Groot et al. (2000) J Org. Chem. 43: 3093 (2000); de Groot et al., (2001) J Med. Chem. 66: 8815; WO 02/083180; Carl et al. (1981) J Med. Chem. Lett. 24: 479; Studer et al. (1992) Bioconjugate Chem. 3 (5): 424-429; Carl et al. (1981) J. Med. Chem. 24 (5): 479-480 and Dubowchik et al. (1998) Bioorg & Med. Chern. Lett. 8: 3347.

Microparticles

The microparticles may be formed of a solid or gel. Suitable gels include polymer gels, for example polysaccharide or polypeptide gels which can be solidified from a liquid into a gel, for example by heating, cooling or pH adjustment. Suitable gels include hydrogels, including alginate, gelatine and agarose gels. Other suitable microparticle materials include inorganic materials such as silicon, glass, metals and ceramics. Other suitable materials include plastics (such as poly(vinyl chloride, cyclo-olefin copolymers, polyacrylamide, polyacrylate, polyethylene, polypropylene, poly(4-methylbutene), polystyrene, polymethacrylate, poly(ethylene terephthalate), polytetrafluoroethylene (PTFE), nylon and polyvinyl butyrate.

Target cells

The assay system of the invention may comprise a target cell. Any suitable target cell may be employed, including prokaryotic and eukaryotic cells.

For example, the target cell may be archaeal, for example selected from the phyla: (a) Crenarchaeota; (b) Euryarchaeota; (c) Korarchaeota; (d) Nanoarchaeota and (e) Thaumarchaeota, for example Haloferax volcanii or Sulfolobus spp..

In other embodiments, the target cell may be a bacterium, for example a pathogenic bacterium. In such cases,

the bacterium may be a Gram-positive bacterium (for example selected from *Enterococcus faecalis, Enterococcus faecium* and *Staphylococcus aureus*), a Gram-negative bacterium (for example selected from *Klebsiella pneumoniae, Acinetobacter baumanii, Escherichia coli, E. coli* ST131 strains, *Pseudomonas aeruginosa, Enterobacter cloacae, Enterobacter aerogenes* and *Neisseria gonorrhoeae*) or may be a bacterium exhibiting an indeterminate Gram reaction.

In other embodiments, the target cell may be a eukaryotic cell, for example selected from: (a) fungal; (b) mammalian; (c) a higher plant cell; (d) protozoal; (e) a helminth cell; (f) algal; and (h) an invertebrate cell. In such embodiments, the target cell may be a cancer cell, for example a human cancer cell, muscle cell, human neuronal cell and other cells derived from a living human patient that show a disease-relevant phenotype.

In cases where the cell is a eukaryotic cell (for example a human cell), the cell may be selected from: totipotent, pluripotent, induced pluripotent, multipotent, oligopotent, stem, embryonic stem (ES), somatic, germ line, terminally differentiated, non-dividing (post-mitotic), mitotic, primary, cell-line-derived and tumour cells.

The cell is preferably isolated (i.e. not present in its natural cellular/tissue *milieu)* and/or metabolically active (for example being present in the assay system along with a culture or transport medium for maintaining cellular viability and/or activity and/or supporting cellular growth or proliferation).

Suitable eukaryotic cells may be isolated from an organism, for example in an organism selected from: metazoan, fungal (e.g. yeast), mammalian, non-mammalian, plant, protozoal, helminth, algal, insect (e.g. fly), fish (e.g. zebrafish), amphibian (e.g. frog), bird, invertebrate and vertebrate organisms.

Suitable eukaryotic cells may also be isolated from non-human animal selected from: mammal, rodent, rabbit, pig, sheep, goat, cow, rat, mouse, non-human primate and hamster. In other embodiments, the cell may be isolated from a non-human disease model or transgenic non-human animal expressing a heterologous gene, for example a heterologous gene encoding a therapeutic product.

Bacteria as target cells

The target cells for use according to the invention may be bacterial cells. In such embodiments, the bacteria may be selected from: (a) Gram-positive, Gram-negative and/or Gram-variable bacteria; (b) spore-forming bacteria; (c) non-spore forming bacteria; (d) filamentous bacteria; (e) intracellular bacteria; (f) obligate aerobes; (g) obligate anaerobes; (h) facultative anaerobes; (i) microaerophilic bacteria and/or (f) opportunistic bacterial pathogens.

In certain embodiments, target cells for use according to the invention may be selected from bacteria of the following genera: *Acinetobacter (e.g. A. baumannii)*; *Aeromonas* (e.g. *A. hydrophila); Bacillus* (e.g. *B. anthracis); Bacteroides* (e.g. B. *fragilis); Bordetella* (e.g. B. *pertussis); Borrelia* (e.g. *B. burgdorferi)*; *Brucella* (e.g. *B. abortus, B. canis, B. melitensis* and B. *suis); Burkholderia* (e.g. *B. cepacia* complex); *Campylobacter* (e.g. *C. jejuni*); *Chlamydia* (e.g. *C. trachomatis, C. suis* and *C. muridarum*); *Chlamydophila* (e.g. (e.g. *C. pneumoniae, C. pecorum, C. psittaci, C. abortus, C. felis and* C. *caviae); Citrobacter* (e.g. *C. freundii*); *Clostridium* (e.g. *C. botulinum, C. difficile, C. perfringens* and *C. tetani*); *Corynebacterium* (e.g. *C. diphteriae* and *C. glutamicum*); *Enterobacter* (e.g. *E. cloacae* and *E. aerogenes*); *Enterococcus* (e.g. *E. faecalis* and *E. faecium*); *Escherichia* (e.g. *E. coli*); *Flavobacterium*; *Francisella* (e.g. *F. tularensis*); *Fusobacterium* (e.g. *F. necrophorum*); *Haemophilus* (e.g. *H. somnus, H. influenzae* and *H. parainfluenzae*); *Helicobacter* (e.g. *H. pylori*); *Klebsiella* (e.g. *K. oxytoca* and *K. pneumoniae*), *Legionella* (e.g. *L. pneumophila*); Leptospira (e.g. *L. interrogans*); *Listeria* (e.g. *L. monocytogenes); Moraxella* (e.g. M. *catarrhalis*); *Morganella* (e.g. *M. morganii*); *Mycobacterium* (e.g. *M. leprae* and *M. tuberculosis*); *Mycoplasma* (e.g. *M. pneumoniae*); *Neisseria* (e.g. *N. gonorrhoeae* and *N. meningitidis*); *Pasteurella* (e.g. *P. multocida*); *Peptostreptococcus; Prevotella; Proteus* (e.g. *P. mirabilis* and *P. vulgaris*), *Pseudomonas* (e.g. *P. aeruginosa*); *Rickettsia* (e.g. *R. nckettsii*); *Salmonella* (e.g. serotypes . Typhi and Typhimurium); *Serratia* (e.g. *S. marcesens); Shigella* (e.g. *S. flexnaria, S. dysenteriae* and *S. sonnei*); *Staphylococcus* (e.g. *S. aureus, S. haemolyticus, S. intermedius, S. epidermidis* and *S. saprophyticus*); *Stenotrophomonas* (e.g. *S. maltophila*); *Streptococcus* (e.g. *S. agalactiae, S. mutans, S. pneumoniae* and *S. pyogenes*); *Treponema* (e.g. *T. pallidum*); *Vibrio* (e.g. *V. cholerae*) and *Yersinia* (e.g. *Y. pestis*).

The target cells for use according to the invention may be selected from high G+C Gram-positive bacteria and from low G+C Gram-positive bacteria.

*Pathogenic bacteria* as *target cells*

Human or animal bacterial pathogens include such bacteria as *Legionella* spp., *Listeria* spp., *Pseudomonas* spp., *Salmonella* spp., *Klebsiella* spp., *Hafnia* spp, *Haemophilus* spp., *Proteus* spp., *Serratia* spp., *Shigella* spp., *Vibrio* spp., *Bacillus* spp., *Campylobacterspp., Yersinia* spp. *Clostridium* spp., *Enterococcus* spp., *Neisseria* spp., *Streptococcus* spp., *Staphylococcus* spp., *Mycobacterium* spp., *Enterobacter* spp.

Fungi as target cells

The target cells for use according to the invention may be fungal cells. These include yeasts, e.g. *Candida* species including *C. albicans, C krusei and C tropicalis,* and filamentous fungi such as *Aspergillus* spp. and *Penicillium* spp. and dermatophytes such as *Trichophyton* spp.

Plant pathogens as target cells

The target cells for use according to the invention may be plant pathogens, for example *Pseudomonas* spp., *Xylella* spp., *Ralstonia* spp., *Xanthomonas* spp., *Erwinia* spp., *Fusarium* spp., *Phytophthora* spp., *Botrytis* spp., *Leptosphaeria* spp., powdery mildews (Ascomycota) and rusts (Basidiomycota).

Cancer cells as targets

Cancer cells may be used as target cells. Such cells may be derived from cell lines or from primary tumours. The cancer cells may be mammalian, and are preferably human. In certain embodiments, the cancer cells are selected from the group consisting of melanoma, lung, renal, colon, prostate, ovarian, breast, central nervous system and a leukaemic cell lines.

Suitable cancer cell lines include, without limitation, ovarian cancer cell lines (e.g. CaOV-3, OVCAR-3, ES-2, SK-OV-3, SW626, TOV-21G, TOV- 112D, OV-90, MDA-H2774 and PA-I); breast cancer cell lines (e.g. MCF7, MDA-MB- 231, MDA-MB-468, MDA-MB-361, MDA-MD-453, BT-474, Hs578T, HCC1008, HCC1954, HCC38, HCCl 143, HCCl 187, HCC1395, HCC1599, HCC1937, HCC2218, Hs574.T, Hs742.T, Hs605.T and Hs606); lung cancer cell lines (e.g. NCI-H2126, NCI- H1395, NCI-H1437, NCI-H2009, NCI-H1672, NCI-H2171, NCI-H2195, NCI-HI 184, NCI- H209, NCI-H2107 and NCI-H128); skin cancer cell lines (e.g. COLO829, TE354.T, Hs925.T, WM-115 and Hs688(A).T; bone cancer cell lines (e.g. Hs919.T, Hs821.T, Hs820.T, Hs704.T, Hs707(A).T, Hs735.T, Hs860.T, Hs888.T, Hs889.T, Hs890.T and Hs709.T); colon cancer cell lines (e.g. Caco-2, DLD-I, HCT-116, HT-29 and SW480); and gastric cancer cell lines (e.g. RF-I). Cancer cell lines useful in the methods of the present invention may be obtained from any convenient source, including the American Type Culture Collection (ATCC) and the National Cancer Institute.

Other cancer cell lines include those derived from neoplastic cells/subjects suffering from neoplasia, including proliferative disorders, benign, pre-cancerous and malignant neoplasia, hyperplasia, metaplasia and dysplasia. Proliferative disorders include, but are not limited to cancer, cancer metastasis, smooth muscle cell proliferation, systemic sclerosis, cirrhosis of the liver, adult respiratory distress syndrome, idiopathic cardiomyopathy, lupus erythematosus, retinopathy (e.g. diabetic retinopathy), cardiac hyperplasia, benign prostatic hyperplasia, ovarian cysts, pulmonary fibrosis, endometriosis, fibromatosis, harmatomas, lymphangiomatosis, sarcoidosis and desmoid tumours. Neoplasia involving smooth muscle cell proliferation include hyperproliferation of cells in the vasculature (e.g. intimal smooth muscle cell hyperplasia, restenosis and vascular occlusion, including in particular stenosis following biologically- or mechanically-mediated vascular injury, such as angioplasty). Moreover, intimal smooth muscle cell hyperplasia can include hyperplasia in smooth muscle other than the vasculature (e.g. blockage of the bile duct, bronchial airways and in the kidneys of patients with renal interstitial fibrosis). Non-cancerous proliferative disorders also include hyperproliferation of cells in the skin such as psoriasis and its varied clinical forms, Reiter's syndrome, pityriasis rubra pilaris and hyperproliferative variants of disorders of keratinization (including actinic keratosis, senile keratosis and scleroderma).

Cell lines as targets

Other cells derived from cell lines may be used as target cells. Such cells, which may preferably be human or mammalian, include those from patients suffering rare diseases with a detectable cellular phenotype. The cells may be of any type, including without limitation blood cells, immune cells, bone marrow cells, skin cells, nervous tissue and muscle cells.

Cell lines useful in the methods of the present invention may be obtained from any convenient source, including the American Type Culture Collection (ATCC) and the National Cancer Institute.

The cells/cell lines may, for example, be derived from subjects suffering from lysosomal storage diseases, muscular dystrophies, cystic fibrosis, Marfan syndrome, sickle cell anaemia, dwarfism, phenylketonuria, neurofibromatosis, Huntington disease, osteogenesis imperfecta, thalassemia and hemochromatosis.

The cells/cell lines may, for example, be derived from subjects suffering from other diseases including diseases and disorders of: blood, coagulation, cell proliferation and dysregulation, neoplasia (including cancer), inflammatory processes, immune system (including autoimmune diseases), metabolism, liver, kidney, musculoskeletal, neurological, neuronal and ocular tissues. Exemplary blood and coagulation diseases and disorders include: anaemia, bare lymphocyte syndrome, bleeding disorders, deficiencies of factor H, factor H-like 1, factor V, factor VIII, factor VII, factor X, factor XI,

factor XII, factor XIIIA, factor XIIIB, Fanconi anaemia, haemophagocytic lymphohistiocytosis, haemophilia A, haemophilia B, haemorrhagic disorder, leukocyte deficiency, sickle cell anaemia and thalassemia.

Examples of immune related diseases and disorders include: AIDS; autoimmune lymphoproliferative syndrome; combined immunodeficiency; HIV -1; HIV susceptibility or infection; immunodeficiency and severe combined immunodeficiency (SCIDs). Autoimmune diseases which can be treated according to the invention include Grave's disease, rheumatoid arthritis, Hashimoto's thyroiditis, vitiligo, type I (early onset) diabetes, pernicious anaemia, multiple sclerosis, glomerulonephritis, systemic lupus E (SLE, lupus) and Sjogren syndrome. Other autoimmune diseases include scleroderma, psoriasis, ankylosing spondilitis, myasthenia gravis, pemphigus, polymyositis, dermomyositis, uveitis, Guillain-Barre syndrome, Crohn's disease and ulcerative colitis (frequently referred to collectively as inflammatory bowel disease (IBD)).

Other exemplary diseases include: amyloid neuropathy; amyloidosis; cystic fibrosis; lysosomal storage diseases; hepatic adenoma; hepatic failure; neurologic disorders; hepatic lipase deficiency; hepatoblastoma, cancer or carcinoma; medullary cystic kidney disease; phenylketonuria; polycystic kidney; or hepatic disease.

Exemplary musculoskeletal diseases and disorders include: muscular dystrophy (e.g. Duchenne and Becker muscular dystrophies), osteoporosis and muscular atrophy.

Exemplary neurological and neuronal diseases and disorders include: ALS, Alzheimer's disease; autism; fragile X syndrome, Huntington's disease, Parkinson's disease, Schizophrenia, secretase related disorders, trinucleotide repeat disorders, Kennedy's disease, Friedrich's ataxia, Machado-Joseph's disease, spinocerebellar ataxia, myotonic dystrophy and dentatorubral pallidoluysian atrophy (DRPLA).

Exemplary ocular diseases include: age related macular degeneration, corneal clouding and dystrophy, cornea plana congenital, glaucoma, leber congenital amaurosis and macular dystrophy.

The cells/cell lines may, for example, be derived from subjects suffering from diseases mediated, at least in part, by deficiencies in proteostasis, including aggregative and misfolding proteostatic diseases, including in particular neurodegenerative disorders (e.g. Parkinson's disease, Alzheimer's disease and Huntington's disease), lysosomal storage disorders, diabetes, emphysema, cancer and cystic fibrosis.

## Archaea as target cells

The target cell may be archaeal, for example selected from the phyla: (a) Crenarchaeota; (b) Euryarchaeota; (c) Korarchaeota; (d) Nanoarchaeota and (e) Thaumarchaeota, for example *Haloferax volcanii* or *Sulfolobus* spp..

Exemplary, archaeal genera include *Acidianus, Acidilobus, Acidococcus, Aciduliprofundum, Aeropyrum, Archaeoglobus, Bacilloviridae, Caldisphaera, Caldivirga, Caldococus, Cenarchaeum, Desulfurococcus, Ferroglobus, Ferroplasma, Geogemma, Geoglobus, Haladaptaus, Halalkalicoccus, Haloalcalophilium, Haloarcula, Halobacterium, Halobaculum, Halobiforma, Halococcus, Haloferax, Halogeometricum, Halomicrobium, Halopiger, Haloplanus, Haloquadratum, Halorhabdus, Halorubrum, Halosarcina, Halosimplex, Halostagnicola, Haloterrigena, Halovivax, Hyperthermus, Ignicoccus, Ignisphaera, Metallosphaera, Methanimicrococcus, Methanobacterium, Methanobrevibacter, Methanocalculus, Methantxaldococcus, Methanocella, Methanococcoides, Methanococcus, Methanocorpusculum, Methanoculleus, Methanofollis, Methanogenium, Methanohalobium, Methanohalophilus, Methanolacinia, Methanolobus, Methanomethylovorans, Methanomicrobium, Methanoplanus, Methanopyrus, Methanoregula, Methanosaeta, Methanosalsum, Methanosarcina, Methanosphaera, Melthanospirillum, Methanothermobacter, Methanothermococcus, Methanothermus, Methanothrix, Methanotorris, Nanoarchaeum, Natrialba, Natrinema, Natronobacterium, Natronococcus, Natronolimnobius, Natronomonas, Natronorubrum, Nitracopumilus, Palaeococcus, Picrophilus, Pyrobaculum, Pyrococcus, Pyrodictium, Pyrolobus, Staphylothermus, Stetteria, Stygiolobus, Sulfolobus, Sulfophobococcus, Sulfurisphaera, Thermocladium, Thermococcus, Thermodiscus, Thermofilum, Thermoplasma, Thermoproteus, Thermosphaera and Vulcanisaeta.*

Exemplary archaeal species include: *Aeropyrum pernix, Archaeglobus fulgidus, Archaeoglobus fulgidus, Desulforcoccus species TOK, Methanobacterium thermoantorophicum, Methanococcus jannaschii, Pyrobaculum aerophilum, Pyrobaculum calidifontis, Pyrobaculum islandicum, Pyrococcus abyssi, Pyrococcus GB-D, Pyrococcus glycovorans, Pyrococcus horikoshii, Pyrococcus spp. GE23, Pyrococcus spp. ST700, Pyrococcus woesii, Pyrodictium occultum, Sulfolobus acidocaldarium, Sulfolobus solataricus, Sulfolobus tokodalii, Thermococcus aggregans, Thermococcus barossii, Thermococcus celer, Thermococcus fumicolans, Thermococcus gorgonarius, Thermococcus hydrothermalis, Thermococcus onnurineus NA 1, Thermococcus pacificus, Thermococcus profundus, Thermococcus siculi, Thermococcus spp. GE8, Thermococcus spp. JDF-3, Thermococcus spp. TY. Thermococcus thioreducens, Thermococcus zilligti, Thermoplasma acidophilum, Thermoplasma volcanium, Acidianus hospitalis, Acidilobus sacharovorans, Aciduliprofundum boonei, Aeropyrum pernix, Archaeoglobus fulgidus, Archaeoglobus profundus, Archaeoglobus veneficus, Caldivirga maquilingensis, Candidatus Korarchaeum cryptofilum, Candidatus Methanoregula boonei, Candidatus Nitrosoarchaeum limnia, Cenarchaeum symbiosum, Desulfurococcus kamchatkensis, Ferroglobus placidus, Ferroplasma acidarmanus, Halalkalicoccus jeotgali, Haloarcula hispanica, Holaoarcula marismortui, Halobacterium salinarum, Halo-*

*bacterium species, Halobiforma lucisalsi, Haloferax volvanii, Halogeometricum borinquense, Halomicrobium mukohataei, halophilic archaceon sp. DL31, Halopiger xanaduensis, Haloquadratum walsbyi, Halorhabdus tiamatea, Halorhabdus utahensis, Halorubrum lacusprofundi, Haloterrigena turkmenica, Hyperthermus butylicus, Igniococcus hospitalis, Ignisphaera aggregans, Metallosphaera cuprina, Metallosphaera sedula, Methanobacterium sp. AL-21, Methanobacterium sp. SWAN-1, Methanobacterium thermoautrophicum, Methanobrevibacter ruminantium, Methanobrevibacter smithii, Methanocaldococcus fervens, Methanocaldococcus infernus, Methanocaldococcus jannaschii, Methanocaldococcus sp. FS406-22, Methanocaldococcus vulcanius, Methanocella conradii, Methanocella paludicola, Methanocella sp. Rice Cluster I (RC-I). Methanococcoides burtonii, Methanococcus aeolicus, Methanococcus maripaludis, Methanococcus vannielii, Methanococcus voltae, Methanocorpusculum labreantum, Methanoculleus marisnigri, Methanohalobium evestigatum, Methanohalophilus mahii, Methanoplanus petrolearius, Methanopyrus kandleri, Methanosaeta concilii, Methanosaeta harundinacea, Methanosaeta thermophila, Methanosalsum zhilinae, Methanosarcina acetivorans, Methanosarcina barkeri, Methanosarcina mazei, Methanosphaera stadtmanae, Methanosphaerula palustris, Methanospiriullum hungatei, Mathanothermobacter marburgensis, Methanothermococcus okinawensis, Methanothermus fervidus, Methanotorris igneus, Nanoarchaeum equitans, Natrialba asiatica, Natrialba magadii, Natronomonas pharaonis, Nitrosopumilus maritimus, Picrophilus torridus, Pyrobaculum aerophilum, Pyrobaculum arsenaticum, Pyrobaculum calidifontis, Pyrobaculum islandicum, Pyrobaculum sp. 1860, Pyrococcus abyssi, Pyrococcus furiosus, Pyrococcus horikoshii, Pyrococcus sp. NA42, Pyrococcus yayanosii, Pyrolobus fumarii, Staphylothermus hellenicus, Staphylothermus marinus, Sulfolobus acidocaldirius, Sulfolobus islandicus, Sulfolobus solfataricus, Sulfolobus tokodaii, Thermococcus barophilus, Thermococcus gammatolerans, Thermococcus kodakaraensis, Thermococcus litoralis, Thermococcus onnurineus, Thermococcus sibiricus, Thermococcus sp. 4557, Thermococcus sp. AM4, Thermofilum pendens, Thermoplasma acidophilum, Thermoplasma volcanium, Thermoproteus neutrophilus, Thermoproteus tenax, Thermoproteus uzoniensis, Thermosphaera aggregans, Vulcanisaeta distributa,* and *Vulcanisaeta moutnovskia.*

Particular examples of archaeal cells useful as producer cells according to the invention include *Haloferax volcanii* and *Sulfolobus* spp.

## Target proteins

The assay system of the invention may comprise a target protein.

Any suitable target protein may be employed, including proteins from any of the target cells discussed in the previous section. Thus, the target protein suitable for use in the assay systems according to the invention may be selected from eukaryotic, prokaryotic, fungal and viral proteins.

Suitable target proteins therefore include, but are not limited to, oncoproteins, transport (nuclear, carrier, ion, channel, electron, protein), behavioural, receptor, cell death, cell differentiation, cell surface, structural proteins, cell adhesion, cell communication, cell motility, enzymes, cellular function (helicase, biosynthesis, motor, antioxidant, catalytic, metabolic, proteolytic), membrane fusion, development, proteins regulating biological processes, proteins with signal transducer activity, receptor activity, isomerase activity, enzyme regulator activity, chaperone regulator, binding activity, transcription regulator activity, translation regulator activity, structural molecule activity, ligase activity, extracellular organisation activity, kinase activity, biogenesis activity, ligase activity, and nucleic acid binding activity.

Target proteins may be selected from, and are therefore not limited to, DNA methyl transferases, AKT pathway proteins, MAPK/ERK pathway proteins, tyrosine kinases, epithelial growth factor receptors (EGFRs), fibroblast growth factor receptors (FGFRs), vascular endothelial growth factor receptors (VEGFRs), erythropoietin-producing human hepatocellular receptors (Ephs), tropomyosin receptor kinases, tumor necrosis factors, apoptosis regulator Bcl-2 family proteins, Aurora kinases, chromatin, G-protein coupled receptors (GPCRs), NF-κB pathway, HCV proteins, HIV proteins, Aspartyl proteases, Histone deacetylases (HDACs), glycosidases, lipases, histone acetyltransferase (HAT), cytokines and hormones.

Specific target proteins may be selected from ERK1/2, ERK5, A-Raf, B-Raf, C-Raf, c-Mos, Tpl2/Cot, MEK, MKK1, MKK2, MKK3, MKK4, MKK5, MKK6, MKK7, TYK2, JNK1, JNK2, JNK3, MEKK1, MEKK2, MEKK3, MEKK4, ASK1, ASK2, MLK1, MLK2, MLK3, p38 α, p38 β, p38 γ, p38 δ, BRD2, BRD3, BRD4, phosphatidyl inositol-3 kinase (PI3K), AKT, Protein kinase A (PKA), Protein Kinase B (PKB), Protein kinase C (PKC), PGC1α, SIRT1, PD-L1, mTOR, PDK-1, p70 S6 kinase, forkhead translocation factor, MELK, eIF4E, Hsp90, Hsp70, Hsp60, topoisomerase type I, topoisomerase type II, DNMT1, DNMT3A, DNMT3B, Cdk11, Cdk2, Cdk3, Cdk4, Cdk5, Cdk6, Cdk7, α-tubulin, β-tubulin, γ-tubulin, δ-tubulin, ε-Tubulin, Janus Kinases (JAK1, JAK2, JAK3), ABL1, ABL2, EGFR, EPH A1, EPHA2, EPHA3, EPHA4, EPHA5, EPHA6, EPHA7, EPHA8, EPHA10, EPHB1, EPHB2, EPHB3, EPHB4, EPHB6, HER2/neu, Her3, Her4, ALK, FGFR1, FGFR2, FGFR3, FGFR4, IGF1R, INSR, INSRR, VEGFR-1, VEGFR-2, VEGFR-3, FLT-3, FLT4,PDGFRA, PDGFRB, CSF1R, Axl, IRAK4, SCFR, Fyn, MuSK, Btk, CSK, PLK4, Fes, MER, c-MET, LMTK2, FRK, ILK, Lck, TIE1, FAK, PTK6, TNNI3, ROSCCK4, ZAP-70, c-Src, Tec, Lyn, TrkA, TrkB, TrkC, RET, ROR1, ROR2, ACK1, Syk, MDM2, HRas, KRas, NRas, ROCK, PI3K, BACE1, BACE2, CTSD, CTSE, NAPSA, PGC, Renin, MMSET, Aurora A kinase, Aurora B kinase, Aurora C kinase, farnesyltransferase, telomerase, adenylyly cyclase, cAMP phosphodiesterase, PARP1, PARP2,

PARP4, PARP-5a, PARP-5b, PKM2, Keap1, Nrf2, TNF, TRAIL, OX40L Lymphotoxin-alpha, IFNAR1, IFNAR2, IFN-α, IFN-β, IFN-γ, IFNLR1, CCL3, CCL4, CCL5, IL1α, IL1β, IL-2, IL-2, IL-4, IL-5, IL-6, IL-7, IL-9, IL-9, IL-10, IL-11, IL-12, IL-13, IL-15, IL-17, Bcl-2, Bcl-xL, Bax, HCV helicase, E1, E2, p7, NS2, NS3, NS4A, NS4B, NS5A, NS5B, NF-κB1, NF-κB2, RelA, RelB, c-Rel, RIP1, ACE, HIV protease, HIV integrase, Gag, Pol, gp160, Tat, Rev, Nef, Vpr, Vif, Vpu, RNA polymerase, GABA transaminase, Reverse transcriptase, DNA polymerase, prolactin, ACTH, ANP, insulin, PDE, AMPK, iNOS, HDAC1, HDAC2, HDAC3, HDAC4, HDAC5, HDAC6, HDAC7, HDAC8, HDAC9, HDAC10, HDAC11, lactase, amylase lysozyme, neuraminidase, invertase, chitinase, hyaluronidase, maltase, sucrase, phosphatase, phosphorylases, P, Histidine decarboxylase, PTEN, histone lysine demethylase (KDM), GCN5, PCAF, Hat1, ATF-2, Tip60, MOZ, MORF, HBO1, p300, CBP, SRC-1, SRC-3, ACTR, TIF-2, TAF1, TFIIIC, protein O-mannosyl-transferase 1 (POMT1), amyloid β and Tau.

Exemplification

The invention will now be described with reference to specific Examples. These are merely exemplary and for illustrative purposes only: they are not intended to be limiting in any way to the scope of the monopoly claimed or to the invention described.

Figure legends

Figure 1: schematic representation of split-and-pool DECL generation. A primary pharmacore is tagged and anchored to a bead *via* a complementary oligonucleotide which is specific for the primary pharmacore. The μM-mM concentrations of guide DNA in the bead yields corresponding μM-mM concentrations of the pharmacore in the bead. Assembly around the primary pharmacore using traditional chemistry methods - each addition is coupled to specific DNA ligated to the end of the encoding tag (which serves as a barcode). The bead can therefore be used for solid-phase synthesis and large quantities of reactive secondary pharmacores can be added for reaction. The ligation of additional oligonucleotide can occur simultaneously (for example *via* click or similar chemistry) or by a post-synthesis step.

Figure 2: schematic representation of split-and-pool DECL. The synthesis step comprises pharmacore (substructure) assembly encoded by DNA sequence (tag) addition as shown in Figure 1. The μM-mM concentrations of guide DNA in the bead yields corresponding μM-mM concentrations of the synthesised chemical structure. The indicator cells are for phenotypic screening. Tag release inside the droplet yields free, tagless chemical structures at concentrations sufficient to interact with the indicator cells in the phenotypic screen. The DNA tags remain spatially associated with the free chemical structures within the droplet. Positive FACs hits are sequenced by NGS sequencing to identify the structure of the chemical structure.

Figure 3: Ligation of encoded oligonucleotides onto bead mounted capture oligonucleotide.

Figure 4: Enzymatic release of a payload from a cleavable linker on a gel.

Figure 5: Enzymatic release of a payload from a cleavable linker in 100 μm aqueous droplets.

Fig 6: Release of a chemical structure payload functionalised with a fluorescent dye using huisgen 1,3-Dipolar cycloaddition from a bead-bound oligonucleotide.

Fig 7: Release of a chemical structure payload functionalised with a fluorescent dye using isothiocyanate addition from a bead-bound oligonucleotide.

Fig 8: Schematic representation of release of free chemical structure using a self-immolative linker.

Fig 9: Schematic representation of split-and-pool DECL using a self-immolative dipeptide linker.

Fig 10: Schematic representation of release of free chemical structures using a Val-Cit-PAB self-immolative peptide linker coupled to beads bearing encoding tag.

Example 1: Generating a tagless compound library using a hydrogel matrix bead with screening in droplets

1: Assembly of guide sequence

1) Assembly involves a 5' tag oligonucleotide which includes a 12C linker to an amine group for crosslinking and a 3' tag oligonucleotide, note the guide sequences are a pool of oligonucleotides corresponding to the monomers for use in the library in this case 200 oligonucleotides allowing for the assembly >1.6 x $10^9$ unique sequences.
2) 5' and 3' tag are added at 1μM final concentration
3) 250 barcode oligonucleotides are pooled at final concentration of 1.25μM
Reaction mixture:

10μl NEBuffer 1 (New England Biolabs)
(1 x reaction mix is 10 mM Bis-Tris-Propane-HCl, 10 mM MgCl2, 1 mM DTT, pH 7 @ 25°C)
5μl 5' tag oligonucleotide @ 100μM
5μl 3' tag oligonucleotide @ 100μM
6.25μl of pooled oligonucleotides @ 100μM
2.5μl Thermostable 5' AppDNA/RNA Ligase (New England Biolabs)
70.25μl of nuclease free water

Incubate for 4h at 65°C

| Oligo. name | Oligo. sequence | 5' modification | 3' modification |
|---|---|---|---|
| 5'tag | ATTATGACCGTAGGCCTTGGC | $NH_2$ - $C_{12}$ linker | None |
| 3' tag | CGC GAT ATT AGC CAT TAA CCC | Adenylated | Final residue is a dideoxy to prevent ligation onto end of tag |
| Pool oligos | Variable 18mers | Adenylated | None |

4) Make up 300μl with 1 x 10mM Tris 1mM EDTA pH7.0 buffer and clean up reaction using an illustra S-200 microspin HR column (GE Healthcare) to eliminate unligated oligonucleotides and reagents.
5) Add 40μl of sodium acetate pH5.2 and 2.5 volumes (1ml) of 100% ethanol. Mix and put at -20°C for at least 1 hour and then pellet DNA by centrifugation at 15,000rpm for 15 minutes at 4°C.
6) Remove supernatant and wash with 1ml of 70% ethanol and spin for 5 minutes, repeat the wash and then air dry the pellet
7) Resuspend the pellet in 25μl of nuclease free water

2: Crosslinking 5' tag oligonucleotide to the agarose to allow crosslinking during PCR

1) Prepare agarose for cross linking by weighing 25g of low melt agarose into 50ml 18.2 ΩM water and mix at 4 °C for 30 minutes to hydrate. Dry by filtering and wash with 100ml of water, recover the slurry and measure the volume (usually 15-20ml).

2) Add an equal volume of 0.05M NaOH to the slurried agarose, confirm pH and adjust to between 10.5 and 11, if need be, by adding 10M NaOH as needed.

3) Place the slurry on a magnetic stirrer and mix as CNBr is added.

4) Add 100mg/ml of slurry Cyanogen Bromide-activated Sepharose (CNBr) (Sigma) (i.e. in 40ml of slurry add 4g of CNBr), immediately check the pH and monitor till the CNBr has fully dissolved (the addition of 1-2ml of 10M NaOH may be required to keep pH between 10.5 and 11). Monitor the pH as reaction proceeds for 15 minutes.

5) After 15 minutes or once the pH becomes static the reaction is complete. Block any remaining active CNBr by adding equal volume of 200mM NaHCO3 at pH 8.5.

6) Using a Buchner funnel filter the slurry and wash 3 x 50ml with 100mM NaHCO3 500mM NaCl at pH8.5.

7) Resuspend in a total volume 25ml of 100mM NaHCO3 500mM NaCl at pH 8.5 buffer.

8) Take 250µl of 5'tag oligonucleotide at 100µM with NH2 - linker on 5' end and mix with the slurried agarose, mix for 2 hours at room temperature.

9) Add equal volume (25ml) of 0.2M Glycine to block any remaining active CNBr binding sites.

10) Filter the slurry and wash with 100ml of 100mM NaHCO3 500mM NaCl at pH8.5 buffer, then wash with 100ml of water air dry and collect gel and weigh (note this material can be kept for storage by adding 0.1% Sodium Azide, alternatively it can be freeze dried and stored at RT for long term usage. If adding Sodium Azide, then the slurry must be filtered and washed to remove azide before usage.

11) This is approximately 40% agarose and is saturated with 5'tag oligonucleotide crosslinked via the amine linker at this stage and can be used later to produce hydrogel matrix. A small aliquot (100µl) is taken and assayed to check it still melts at roughly 75°C and sets solid below 20°C to check crosslinking has not adversely affected gelling properties.

3: <u>PCR in beads</u>

This step amplifies up, in a single droplet, a single specific DNA guide, thus yielding a clonal DNA population in each bead. Note the cross linked 5'tag oligonucleotide is used in the PCR, but remains bound to the agarose as covalent linkage. This cannot be lost from the agarose. This gives a clonal bead with µM concentrations of DNA guide attached all over and within the bead.

1) Either dissolve dried agarose-CNBr-oligonucleotide or add slurry to a final concentration of 0.5% as needed.

2) Make the aqueous reaction mixture as follows and keep it warm. The reduced 3'Rev tag favours production of 5'tag product by asymmetric PCR, thus more cross-linked guide is produced compared to the template. Assembled guide DNA is at 1 copy/droplet (some droplets are empty)
Reaction make up 1ml volume/encapsulation

200µl 5 X Phusion HF buffer
20µl 10mM dNTP mix (equal mix)
2µl of 3' Rev-comp oligonucleotide (GGGTTAATGGCTAATATCGCG)
50µl Phusion HSII polymerase (New England Biolabs)

To 1000µl with nuclease free water with 0.015g of CNBr-5' tag agarose (so 1.5% agarose beads).

3) Encapsulate in a 14 µm etched droplet generation chip (Dolomite, UK) using PicoSurf 1 (2 % surfactant, Dolomite, UK) as the continuous phase (20 µL/min aqueous phase, 40 µL/min oil phase.

4) PCR the complete droplet mix in bulk - split between 48 wells (50µl/well).

5) 98°C 2minutes [98°C 15 seconds, 54°C 15 seconds, 72°C 10 seconds] x 30 72°C 2 minutes then to 4°C for 20 minutes to solidify (keep at 4°C until ready to proceed).

6) Pool beads and add 5 volumes (5ml) of PBS + 1 % Tween80 and mix by inversion, centrifuge at 2500g for 15minutes at 4°C to pellet.

7) Remove supernatant and wash beads with 5 volumes (5ml) PBS + 1 % Tween80 again to remove all oil, centrifuge at 2500g for 15 minutes at 4°C to pellet.

8) Beads are now free of oil, to remove any hybridised and not crosslinked DNA (or reverse complement to guide)

wash with 5ml of 0.1M NaOH, mix at RT for 5 minutes, then centrifuge at 2500g for 15minutes at 4°C to pellet.

9) Repeat 0.1M NaOH wash

10) Add 5ml of water to the beads and centrifuge at 2500g for 15minutes at 4°C to pellet.

11) Repeat water wash, beads are now ready to guide chemistry reaction

4: Guided assembly of chemical library

DNA guides assembly of monomers - were synthesised using conventional chemistry the RNA tags were synthesised in advance by IDT. The last 3 nucleotides contain phosphorothioate linkages to prevent cleavage by exonucleases. Individual reagents are then tagged with a guide to a specific RNA specific to a unique sequence incorporated into the guide oligonucleotide (100 unique monomers can be assembled, assuming up to 4 guide sequences into over 100,000,000 unique compounds). Guides and tags are between 18 and 25mers and with a Tm for DNA of >60°C however as RNA-DNA interactions are more stable we have seen real melting temperatures of 75-85°C for these oligonucleotides.

1) Beads are packed into a column (each bead is clonal with a unique DNA sequence to guide assembly but μM concentrations of this clonal guide on each bead) and at 20μM 200 million can be present per ml of bead volume.

2) A pool of monomers and appropriate reagents are then washed onto the column and reactions allowed to proceed, the column can be washed and new reaction chemistry added as needed (note fresh monomers can also be added).

3) Chemical synthesis varies with monomers used (see example 2, section 4).

4) Finally the beads are washed with 10 column volumes of TE with 100mM NaCl and then 2 column volumes of appropriate media for growth of cells in the phenotypic screen e.g. RPMI.

5) Beads are then collected in the growth media and ready for encapsulation with target cells. The newly synthesised compound remains bound *via* the DNA guide - RNA tag interaction (as long as pH kept 5-9 and temperature below 75°C (as the new compound is attached *via* 2-4 guides it is very stable).

5: Encapsulation of chemical bead with indicator cells

Chemicals on bead remain bound until encapsulation. Jurkat cells are diluted so as to trap 2-8/droplet.

1) The agarose beads, 2-8 Jurkat cells in RPMI media and 0.4 % w/v type IX-A agarose and 0.1μg/ml RNaseA are encapsulated at 100 μL/min aqueous flowrate and 200 μL/min with Picosurf 1 (Dolomite, uk) in a droplet generation chip with a 100 μm junction. < 1 agarose bead is encapsulated per droplet to maintain clonality.

2) Chemical release from the hybridised compounds is due to incorporation of 0.1μg/ml RNaseA in the cell mixture. This doesn't affect cellular growth and is active for at least 48h in growth media tested. Cleavage of RNA guides occurs within 15-20 minutes in RPMI, lysogeny broth, tryptic soy broth and DMEM. RNaseA cleaves the RNA tag (but not the DNA) leaving and untagged compound no longer bound to the guide and free to diffuse out of the agarose bead and into the indicator droplet. This occurs at 0.5-100μM concentrations.

6: Phenotypic screening

1) After incubation of chemical bead with indicator cell for 24h the droplets are broken. It is possible to incubate the cells for longer periods: for example, human cells can be incubated in assays for 10 days and bacterial cells are recovered after 28. However, usual incubation times are 24-144h.

2) Beads are solidified by putting on ice, then broken by adding 4 volumes of Phosphate Buffered Saline (PBS) + 1% Tween80, mixing by inversion and then centrifugation at 3500g for 15 minutes at 4°C

3) Supernatant is removed and the bead pellet washed again with 4 volumes PBS + 1% Tween80.

4) Bead pellet is then filtered through 125μm onto a 45μm particle filter, this removes larger droplets and washed through smaller debris and cells.

5) The beads on the filter are rinsed with 5 volumes of PBS and then collected in a centrifuge tube.

6) Make up mixture to 100ml with PBS

7) Add TMRM and Hoescht 33342 (Thermo Fisher Scientific) dyes at 200μM final concentration and 1μg/ml respectively. Incubate at room temperature for 30 minutes.

8) Pellet beads by centrifugation at 3500g for 15 minutes at 4°C.

9) Resuspend in 50ml PBS and repeat wash step to remove dye

10) Using the Fluorescent Activated Cell Sorter (FACS) excite the Hoescht using 405nm laser and the TMRM using 488nm laser, detect output of these channels compared to the known controls.

11) Any droplets showing increased TMRM/Hoescht stain ratio are sorted into a tube

7: Sequencing targets to identify hit compounds

1) Pellet 'hit' beads and resuspend in 25μl of water

2) Set up 1st round PCR to increase copies of each hit - noted millions per droplet attached to agarose by long linker.

10μl of HF buffer (Thermo Fisher Scientific)
1μl of 5'tag at 10μM (no linker version ATTATGACCGTAGGCCTTGGC)
1μl 3' Rev-comp oligonucleotide at 10μM (GGGTTAATGGCTAATATCGCG)
0.25μl Phusion DNA polymerase (Thermo Fisher Sceintific)
25μl of beads in water
12.75μl of nuclease free water to bring to 50μl

PCR 98°C 2 minutes [98°C 15 seconds, 54°C 15 seconds, 72°C 10seconds] x 15 72°C 2 minutes

3) Clean up with Ampure XP beads (Beckman Coulter)

- Add 1.8 volumes (90μl of Ampure XP beads) mix well by pipetting
- Wait 2 minutes and then place on magnet, remove supernatant once beads have collected 2-5 minutes
- Wash with 200μl of 80% ethanol, incubate at room temperature for 2'
- Place back on the magnet for 2-5 minutes and remove ethanol
- Repeat ethanol wash this time air dry for 5 minutes until the beads are dry
- Resupend in 20μl of 10mM Tris-HCL pH7.0 this elutes DNA and place back on magnet
- Collect and keep supernatant

4) Second round PCR to add primers

5) Using 5' and 3' tag and modified RC_INDEX and PS_12 index primers from illumina to encode (12 x EC and 8 x PS_I2) with the tag sequences on the 3' end of the primer, these tags are constant but index changes to encode 96 well plate PCR with following conditions:

- 10μl 2 x NEBNext PCR master mix (New England Biolabs)
- 2.5μl of RC_INDEX at 3.3μM (1 in 30 dilution of stock)
- 2.5μl of PS_12 primer at 3.3μM (1 in 30 dilution of stock)
- 5μl of DNA eluted from PCR one (step 3)
- PCR 98°C 2minutes [98°C 10 seconds, 54°C 60 seconds, 72°C 30seconds] x 30 72°C 2 minutes

6) Size selection of PCR products selectin 250-350bp using Ampure XP beads (Beckman Coulter). To remove larger fragments:

- Add 15μl of water to increase volume to 65μl.
- Add 49μl (0.75x volumes) of Ampure beads and mix well, incubate for 5 minutes.
- Place on magnet for 2 minutes and transfer supernatant to a clean well.
- To bin desired fragments
- Add 10μl (0.15x volumes of Ampure beads and mix well, incubate for 5 minutes.
- Place on magnet and this time discard the supernatant.
- Wash with 200μl of 80% ethanol, incubate at room temperature for 2 minutes.
- Place back on the magnet for 2-5 minutes and remove ethanol.
- Repeat ethanol wash this time air dry for 5 minutes until the beads are dry.
- Resupend in 35μl of 10mM Tris-HCL pH7.0 this elutes DNA and place back on magnet.
- Collect and keep 25μl of supernatant.

7) Set up dilutions 1:100. 1:10000 and 1:200000. Using Kapa Fast qPCR (Kapabiosytems) for Illumina quant kit in 12μl reactions determine DNA concentration by qPCR.

8) Using qPCR results create a 2nM library mix of all samples and load onto sequencer following the Illumina protocols for NextSeq500 system guide (15046563) and NextSeq Denature and Dilute Libraries Guide (15048776).

9) Returned sequences of guide nucleic acid flagged by the two tags are easily identified and used to determine the order of compound assembly and its structure - the guide, being less than 200 nucleotides, can be synthesised using ultramers from IDT if required to make more compound from monomers this way quickly and confirm activity. Alternatively, the compound is synthesised directly based on structure decoded from DNA sequencing data.

Example 2 - Generating a tagless compound library using an immobilised DNA Guide on solid beads

1: Assembly of guide sequence

As described in Example 1, section 1, except the 5' tag will be biotinylated rather than an amine.

2: Attaching a single guide to a streptavidin coated bead

To attach a single DNA molecule to an activated bead coated in streptavidin to allow attachment. Beads were purchased from Bangs laboratories Cat No: CP01N with mean diameter of 4.95μm. Beads are polymer coated in streptavidin.

1) 1000μl (5mg of beads) were taken and pelleted by centrifugation 5000g for 5' at room temperature.

2) Supernatant was removed and the beads were washed with 1000μl of 100mM Tris-HCl pH8, 0.1% Tween20

3) Wash was repeated 2 x more to remove any remaining buffer

4) Resuspend the beads in 100μl final volume of 100mM Tris-HCl pH8, 0.1% Tween20

5) Beads are 6 x $10^7$ beads/mg so in 1000μl we have 6 x 108 beads meaning we need the same number of guides to get on average one per bead. To this end we need roughly 3femto moles of DNA, as the average guide is 123bp in length this means we need 0.25ng of oligonucleotide DNA empirically we have found we need roughly twice this to get best results so use 0.4ng of DNA guide construct - this is diluted into 100μl of 100mM Tris-HCl pH8, 0.1% Tween20 and mixed with the beads.

6) The beads are mixed at room temperature for 1 hour

7) Beads are pelleted by centrifugation and are washed 5 x with 500μl of 100mM Tris-HCl pH8, 0.5% Tween20 to remove unbound DNA as described in steps 1 and 2

8) Final resuspension is in 500μl of 10mM Tris-HCl pH8, 1mM EDTA buffer and can be stored for weeks at 4°C,

10μl are run on a 0.5% agarose gel and stained with 1 x sypro-ruby to stain protein on bead and 1 x Sybr Gold to stain the DNA, should see both localised although SybR gold can be quite weak on gel and up to 4 distinct bands (1 - 4 variable regions in guide) with the majority running as the larger fragment. As attached to beads fragments appear to be >10kb in all cases. If more bands are visible the batch is not homogenous and so has to be repeated.

9) Samples are diluted as droplets are made to give one bead per droplet.

3: Amplification of guide oligonucleotide on functional beads

1) Dilute the oligonucleotide functionalised beads to 70,000 beads/uL in the following mixture:
Reaction make up 1ml volume/encapsulation:

200μl 5 X Phusion HF buffer (Thermo Fisher Scientific)
20μl 10mM dNTP mix (equal mix)
2μl of 3' Rev-comp oligonucleotide (GGGTTAATGGCTAATATCGCG) (IDT)
50μl Phusion HSII polymerase (Thermo Fisher Scientific)

2) Encapsulate in a 14 μm etched droplet generation chip (Dolomite, UK) using Pico-Surf 1 (2 % surfactant, Dolomite, UK) as the continuous phase (20 μL/min aqueous phase, 40 μL/min oil phase. The bead dilution ensures one bead every 10 droplets, guaranteeing clonality and takes roughly 1 hour to encapsulate the full mixture).

3) Collect the resulting 14 μm droplets (18 pL) in an Eppendorf tube.

4) PCR the complete droplet mix in bulk - split between 60 wells (50μl/well).

5) 98°C 2 minutes [98°C 15 seconds, 54°C 15 seconds, 72°C 10 seconds] x 30 72°C 2 minutes then to 4°C for 20 minutes to solidify (keep at 4°C until ready to proceed).

6) Pool beads and break the droplets using Pico-break 1 (Dolomite, UK) following the manufacturers protocol.

7) Recover the beads and wash beads with 5 volumes (5ml) PBS + 1 % Tween80 again to remove all oil, centrifuge at 2500g for 15 minutes at 4°C to pellet.

8) Beads are now free of oil, to remove any hybridised and not crosslinked DNA (or reverse complement to guide) wash with 5ml of 0.1M NaOH, mix at RT for 5 minutes, then centrifuge at 2500g for 15 minutes at 4°C to pellet.

9) Repeat 0.1M NaOH wash.

10) Add 5ml of water to the beads and centrifuge at 2500g for 15 minutes at 4°C to pellet.

11) Repeat water wash, beads are now ready for use.

4: DNA templated assisted synthesis on beads

The assembly of the tagged monomers for DNA directed assembly were synthesised using conventional chemistry. The RNA tags were synthesised in advance by IDT and the last 3 nucleotides contained phosphorothioate linkages to prevent cleavage by exonucleases. Individual reagents are then tagged with a guide to a specific RNA specific to a unique guide sequence incorporated into the guide oligonucleotide (100 unique monomers can be assembled, assuming up to 4 guide sequences into over 100,000,000 unique compounds). Guides and tags are designed between 18 and 25mers and with a Tm for DNA of >60°C however as RNA-DNA interactions are more stable we have seen stability to 75-85°C for these oligonucleotides. Monomer concentrations are kept below 0.5 μM to minimise cross reactivity. Reagents were linked to the 5' end of a sequence corresponding the pools of guide oligonucleotides. In this illustrative example, building blocks containing either (a) Aldehydes or (b) amines are used to link monomers together resulting in a secondary amine linkage after reductive amination.

1) Dilute functionalised guidance beads (from section X, Example 2) to 20,000 beads/μL in 50 mM TAPS buffer,

pH 8.0, with 250 mM NaCl, 10 mM sodium borohydride and 0.5 μM reactive oligonucleotide linked monomers.

2) Using a microfluidic chip with a 20 μm etched depth (Dolomite, UK), encapsulate this mixture with an aqueous flow rate of 50 μL/min and 100 μL/min Pico-surf 1 (2 % surfactant, Dolomite, UK) resulting in 20 μm diameter droplets (42 pL).

3) Collect droplets into a 50 mL Falcon tube, and allow to react at 25 °C for 24 hours.

5: Droplet merging of assembled compound, guidance bead and indicator cells

This step involves a custom made microfluidic chip containing a small droplet generation site, a separate larger droplet generation site for making droplets 100 μm in diameter, a Y-shaped channel for droplet synchronisation and a pair of addressable electrodes for droplet coalescence. These devices are readily available from companies such as Dolomite, UK or Micronit, NL.

1) Prepare a mixture containing the following for the indicator droplets:

Jurkat cells (21,000 cells/uL for 5 cells/droplet
RNase A, 0.1 μg/mL
0.5 % wt Type IX-A agarose (Sigma)
RPMI media

2) Begin reinjection of the 20 μm droplets from step 4, example 2 in the custom made chip.

3) Begin making 100 μm droplets using a flow rate of 100 μL/mL, with a Pico-surf 1 flow rate of 200 μL/min.

4) Using a high speed camera (Pixellink, Canada) match the speed of reinjection of the 20 μm droplets to the generation rate of the 100 μm droplets to ensure 1:1 synchronisation at the Y-channel. The smaller droplets experience less hydrodynamic drag in the channel and move faster, catching up with the 100 μm droplets and self-synchronising into pairs.

5) Merge the contiguous pairs of droplet by electrofusion at the electrodes by applying a an electric field of 4 kV/cm with square wave pulses at 1 KHz from a pulse generator (Aim-TTi, RS components, UK) and a high voltage amplifier (Trek 2220, Trek, USA).

6) Collect the merged droplets off chip, incubate in a tissue culture flask (Fisher, UK) at 37 °C with 5 % CO2.

6: Phenotypic screening

As described in Example 1, section 6.

7: Sequencing targets to identify hit compounds

As described in Example 1, section 7.

Example 3 - Reversibly tagging an existing compound library

1: Generation of random oligonucleotide tags for addition to compound library

1) Random tags are purchased from Twist Bioscience comprising 100,000-1,000,000 unique sequences 18-25mers with a Tm >55°C, to each is added a 5'
RNA_Adaptor1 oligonucleotide and 3' DNA_Adaptor1 oligonucleotide each is modified to ensure addition to specific end.
2) Reaction mixture for 200μl reaction:

20μl 10 X Buffer (1 X Reaction Buffer (50 mM Tris-HCl, pH 7.5, 10 mM MgCl2, 1 mM DTT)
100μl 50% PEG 8000 (w/v) (1 x 25% (w/v) PEG 8000)
20μl 10mM hexamine cobalt chloride (1 x 1 mM hexamine cobalt chloride)

20 μl (100 units) T4 RNA Ligase
20μl 10mM ATP (1 x 1 mM ATP)

20μl oligonucleotide mix (equal mix of the random barcode oligonucleotides and the RNA and DNA adaptor at 30μM each in water). Incubate at 25°C for 16 hours. Stop the reaction by adding 40 μl 10 mM Tris-HCl pH 8.0, 2.5 mM EDTA.

| Oligo. name | Oligo. sequence | 5' modification | 3' modification |
|---|---|---|---|
| 5' DNA_Adaptor1 | ATTATGACCGTAGGCCTTGGC | None | None |
| 3' RNA_Adaptor1 | CGC GAT ATT AGC CAT TAA CCC | phosphorylated | None |
| Barcode oligo. | Variable 18-25mers | Phosphorylated | None |
| Capture oligo. | GGGTTAATGGCTAATATCGCG | $NH_2$ - $C_{12}$ linker | |

3) Make up 300μl with 10mM Tris, 1mM EDTA buffer, pH7 and clean up reaction using an illustra S-200 microspin HR column (GE Healthcare) to eliminate unligated oligonucleotides and reagents.
4) Add 40μl of sodium acetate pH5.2 and 2.5 volumes (1ml) of 100% ethanol. Mix and put at -20°C for at least 1 hour and then pellet DNA by centrifugation at 15,000rpm for 15minutes at 4°C.
5) Remove supernatant and wash with 1ml of 70% ethanol and spin for 5minutes, repeat the wash and then air dry the pellet.
6) Resuspend the pellet in 25μl of nuclease free water

2: Crosslinking 5' tag oligonucleotide to the agarose to allow crosslinking during PCR

1) Prepare agarose for cross linking by weighing 25g of low melt agarose (Sigma) into 50ml 18.2ΩM water and mix at 4 °C for 30 minutes to hydrate. Dry by filtering and wash with 100ml of water, recover the slurry and measure the volume (usually 15-20ml).
2) Add an equal volume of 0.05M NaOH to the slurried agarose, confirm pH and adjust to between 10.5 and 11 if need be by adding 10M NaOH as needed.
3) Place the slurry on a magnetic stirrer and mix as CNBr (Sigma) is added.
4) Add 100mg/ml of slurry CNBr i.e. in 40ml of slurry add 4g of CNBr, immediately check the pH and monitor till the CNBr has fully dissolved you may need to add 1-2ml of 10M NaOH to keep pH between 10.5 and 11 units. Monitor the pH as reaction proceeds for 15minutes.
5) After 15 minutes or once the pH becomes static the reaction is complete. Block any remaining active CNBr by adding equal volume of 200mM $NaHCO_3$ at pH8.5.
6) Using a Buchner funnel filter the slurry and wash 3 x 50ml with 100mM $NaHCO_3$ 500mM NaCl at pH8.5.
7) Resuspend in a total volume 25ml of 100mM $NaHCO_3$ 500mM NaCl at pH8.5 buffer
8) Take 250μl of 5'tag oligonucleotide at 100μM with $NH_2$ - linker on 5' end and mix with the slurried agarose, mix for 2h at room temperature
9) Add equal volume (25ml) of 0.2M Glycine to block any remaining active CNBr biding sites.
10) Filter the slurry and wash with 100ml of 100mM $NaHCO_3$ 500mM NaCl at pH8.5 buffer, then wash with 100ml of water air dry and collect gel and weigh (note this material can be kept for storage by adding 0.1% Sodium Azide, alternatively it can be freeze dried and stored at RT for long term usage. If adding azide, the slurry to be must be filtered and washed to remove azide before usage.
11) This is approximately 40% agarose and is saturated with 5' tag oligonucleotide crosslinked via the amine linker at this stage and can be used later to produce hydrogel matrix. A small aliquot (100μl is taken and assayed to check it still melts at roughly 75°C and sets solid below 20°C to check crosslinking has not adversely affected gelling properties.

3) Tagging the compound library (chemical method)

The exact nature of this reaction will depend on the library in question and the functional groups contained within, but in this example the library is heavily functionalised with amino groups. The barcoded oligonucleotide is then

functionalised at the terminal end with a succinimidyl ester. The barcoded oligonucleotide is functionalised with a cleavable group in order to release the compound within the droplet.

(1) Dissolve the individual library members in 0.1 M sodium bicarbonate buffer, pH 8.3 to a final concentration of 1 mM.
(2) Add succinimidyl ester functionalised guide oligonucleotide to the individual wells to achieve a 1:1 molar ratio. Ensure only one specific sequence is added to each individual well.
(3) Incubate for one hour at room temperature
(4) Warm the solution to 40 °C
(5) Dilute the stock of beads from step 3 and load into the well. Aim to load at least 100 beads/well to ensure oversampling during the screening process.
(6) Incubate at 40 °C to hybridise the tagged compound to the beads.
(7) Spin down remove the supernatant from each well. Wash three times with 1XPBS buffer.
(8) Wash the beads out of the wells using 1X PBS buffer. Pool together, then resuspend in fresh 1XPBS.

4) Tagging the compound (enzymatic method)

Again the exact nature of this reaction and functional groups, essentially any suitable enzyme can be used including glycosylases, none ribosomal peptide synthase, ubiquitinase, and phosphorylases to name a few. In this description we use a phosphorylase enzyme.

Dissolve the compound in water or DMSO to 0.5 mM (no more than 2.5 μL if in DMSO):

(1) 5μl of 10 x reaction buffer (final is 70 mM Tris-HCl, 10 mM $MgCl_2$, 5 mM DTT pH 7.6 @ 25°C
(2) 10μl 50% (w/v) PEG-4000 (finale concentration 10%)
(3) 2.5μl 10mM ATP (Final concentration 500μM)
(4) 5μ T4 PNK (New England Biolabs)
(5) Make up to 50μl with water

Incubate at 37°C for 4h

Random tags are purchased from Twist Bioscience comprising 100,000-1,000,000 unique sequences 18-25mers with a Tm >55°C, to each is added a 5' DNA_Adaptor1 oligonucleotide and 3' RNA_Adaptor1 oligonucleotide each is modified to ensure addition to specific end. This reaction ligates the barcode together.

Make the following mix for 200 μL:

(1) 20μl 10 X Buffer (1 X Reaction Buffer (50 mM Tris-HCl, pH 7.5, 10 mM MgCl2, 1 mM DTT)
(2) 100μl 50% PEG 8000 (w/v) (1 x 25% (w/v) PEG 8000)
(3) 20μl 10mM hexamine cobalt chloride (1 x 1 mM hexamine cobalt chloride)
(4) 20 μl (100 units) T4 RNA Ligase (New England Biolabs)
(5) 20μl 10mM ATP (1 x 1 mM ATP) (Sigma)
(6) 20μl oligonucleotide mix (equal mix of the random barcode oligonucleotides and the RNA and DNA adaptor at 30μM each in water)

Incubate at 25°C for 16 hours.

Stop the reaction by adding 40 μl 10 mM Tris-HCl pH 8.0, 2.5 mM EDTA.

| Oligo. name | Oligo. sequence | 5' modification | 3' modification |
|---|---|---|---|
| 5' DNA_Adaptor1 | ATTATGACCGTAGGCCTTGGC | None | None |
| 3' RNA_Adaptor1 | CGC GAT ATT AGC CAT TAA CCC | phosphorylated | None |
| Barcode oligo. | Variable 18-25mers | Phosphorylated | None |

1) Make up 300μl with 10mM Tris, 1mM EDTA pH7 buffer and clean up reaction using an illustra S-200 microspin HR column (GE Healthcare) to eliminate unligated oligonucleotides and reagents.
2) Add 40μl of sodium acetate pH5.2 and 2.5 volumes (1ml) of 100% ethanol. Mix and put at -20°C for at least 1

hour and then pellet DNA by centrifugation at 15,000rpm for 15minutes at 4°C.

3) Remove supernatant and wash with 1ml of 70% ethanol and spin for 5minutes, repeat the wash and then air dry the pellet

4) Resuspend the pellet in 25μl of nuclease free water

5) Attaching the compound to the beads

1) To above compound add 50μl of generic attachment beads with NH-$C_{12}$-GGGTTAATGGCTAATATCGCG oligonucleotide (as described in Example 3, section 2).

6) Phenotypic screening

As described in Example 1, section 6.

7) Sequencing targets to identify hit compounds

As described in Example 1, section 7.

Example 4 - Split-pool method for tagless DECL synthesis

The widely-used split-and-pool strategy (see e.g. Mannocci et al. (2011) Chem. Commun., 47: 12747-12753; Mannocci et al. (2008) Proc. Natl. Acad. Sci. 105: 17670-17675) can be used to generate a single-pharmacophore DECL for use according to the invention based on the stepwise split-&-pool combinatorial assembly of multiple sets of chemical substructures and corresponding DNA-coding fragments involving iterative chemical synthesis and DNA encoding steps, as described below.

1) Overview

Referring now to Figure 1, guide oligonucleotide is first attached to functionalised agarose beads, each bead containing at least $10^{11}$ functional groups for oligonucleotide attachment. A tagged substructure (which may be referred to herein as a "pharmacore") is linked *via* a cleavable linker to an oligonucleotide complementary to the guide oligonucleotide and then attached to the guide oligonucleotide by ligation. This serves as the primary pharmacore which is then "decorated" with further pharmacores (secondary, tertiary, etc pharmacores), as explained below).

Consecutive rounds of synthesis are then carried out to decorate the primary pharmacore substructure by reaction with further substructures/pharmacores. A coding oligonucleotide for each reaction and building block is also added to the guide oligonucleotide by ligation. The library is constructed using a split and pool technique with alternative rounds of chemical synthesis and coding ligation. After library generation, each bead is decorated with multiple copies of a single compound.

Referring now to Figure 2, the library beads are then encapsulated inside microdroplets with indicator cells (a protein target could also be used). The compound is released by addition of enzymes that cleave the cleavable linker within the droplet releasing the compound into the aqueous interior. The droplets are then incubated for a predetermined period and the phenotypic effect of the compound determined using FACS. Droplets in which the cells show the desired phenotypes are sorted and collected. The oligonucleotide that encodes the library member synthesis is therefore spatially, but not physically linked to the phenotypic effect it causes.

Compound identification is carried out by cleaving the guide oligonucleotide by restriction digestion from the bead followed by sequencing. The coding sequence of oligonucleotide describes the synthetic steps that pharmacore was exposed to and the structure of the compound responsible for the phenotypic effect observed by FACS. PCR amplification of the guide oligonucleotide is not needed as >$10^{10}$ oligonucleotide molecules are present, although PCR can be used to amplify if required.

This tagged library synthesis retains the key advantages of DNA encoding (barcodlng) and scale of synthesis, yet allows the synthesis of molecule libraries using small number of relatively expensive tagged chemical substructures. It also reduces any scar to that arising from a single cleavage event. The split and pool method therefore allows diverse pharmacore addition utilising known chemistry, since there is no need for all the pharmacore substructure units to be tagged.

2) Functionalisation of agarose amine beads

A 2 mL aliquot of a 50 % suspension of amine functionalised agarose beads (Cube Bioscience) was spun down at 500xg for 10 mins and washed 3x with RO water, then suspended in 5 mL of 100 mM MES + 150 mM NaCl coupling buffer, pH 5.5. In separate tubes, an azide functionalisation mix was prepared. 2.5 mL of a 20 mM solution of N-hydroxysucciminde (NHS, Sigma Aldrich) and 2.5 mL of a 20 mM solution of N-(3-Dimethylaminopropyl)-N'-ethylcarbodiimide hydrochloride (EDC, Sigma Aldrich), both in coupling buffer, were mixed. 2-Azidoacetic acid (5 μL, Carbosynth) was added, and this mix was left to react for 20 mins at room temperature. After 20 mins, the amine beads were spun down and resuspended in 5 mL of the azide functionalisation mix. The tube was placed on a rotator for one hour at room temperature, then spun down and resuspened in 5 mL of fresh azide functionalisation mixture. This was placed on a rotator and allowed to react overnight. The beads were then washed with 3 x 5 mL of 1XPBS, pH 7.4, then resuspended in 5 mL of 100 mM sodium hydrogen carbonate pH 7.5 and stored at 4 °C until needed.

3) Attachment of capture oligonucleotide to functionalised azide-agarose beads

1 mL of the azide bead stock was spun down and washed with 3 x with 1 mL of 100 mM MOPS, pH 7.0. The beads were then suspended in 785 μL of MOPS buffer. 100 uL of capture oligonucleotide (5'-GAAGGGTCGACTAAG ATTATACTGCATAGCTAGGGGAATGGATCCCGCC TTTTTTT(Int 5-Octadiynyl Du)TTTTTTTTT GGCGGGATCC-3', ADTbio, 48.92 μM) was added, followed by 10 μL of a 50 mM tris(3-hydroxypropyltriazolylmethyl)amine (THPTA, Sigma Aldrich), 5 μL of a 20 mM solution of copper (II) sulphate (Sigma Aldrich) and 100 μL of a 10 mM solution of sodium ascorbate (Sigma Aldrich). The beads were placed on a rotator and left to react overnight, then the mix was spun down and washed with 1 x mL 100 mM MOPS buffer, then resuspended in 758 μL MOPS buffer, 100 μL of 10 mM Propargyl alcohol (Sigma Aldrich), followed by 10 μL of a 50 mM THPTA, 5 μL of a 20 mM solution of copper (II) sulphate and 100 μL of a 10 mM solution of sodium ascorbate. After one hour, the beads were washed with 3 x 1 mL of MOPS buffer, then resuspended in 100 mM sodium hydrogen carbonate buffer, pH 7.5.

4) Ligation of cleavable oligonucleotide with primary pharmacore

1 ml of the azide agarose beads + capture oligonucleotide was spun down and washed 3x with 500μL of nuclease free water (NFW) before being resuspended in 475 μL of NFW and 500 uL 2x Quick Ligase Buffer (NEB). 15 μL of the payload oligonucleotide was added (5'ATTCCCCTAGCTATGCAAGTrGrArGRrArArGrUX3', Where X = hexynyl hydrodroxyprolinol, ADTbio, 81.53 μM) with 10 μL Quick Ligase. The beads were incubated with rotation at 37 °C, then washed 3X with 100 mM sodium hydrogen carbonate buffer, pH 7.5 and stored at 4°C until needed.

5) Tagging

Tagging by ligation of encoding oligonucleotides to the capture oligonucleotide on beads was performed by washing the beads into water (centrifuge beads at 5000g, 5minutes, resuspend in 5 x bead volumes of nuclease free water (NFW) repeat wash for a total of 2 times. The beads then resupended in 5 x bead volume of ligation mixture which has a final concentration of 1 x Quick ligase buffer (New England Biolabs), 250nM Splint oligonucleotide, 250nM encoding oligonucleotide, 20U/μl T4 DNA ligase (New England Biolabs) and then nuclease free water to 5 x bead volume).

To test ligation efficiency and maximise efficiency of ligation, the samples split into two. One sample was heated to 95°C for 2 minutes then cooled to 4°C in thermal cycler at 0.2°C/second. The other was sample was not heat treated and left at room temperature. T4 DNA ligase (20U/μl) was added after the heat step.

Both reactions were incubated for 1h at 37 °C to allow ligation to proceed. Once incubated the ligase was heat inactivated by heating to 95°C for 20minutes then the oligonucleotide was cut from the beads using 20U/μl BamHI (NEB) and incubated for 1h further at 37°C. Beads were pelleted by centrifugation (5000g, 5minutes) and then supernatant taken off. This DNA cute from the beads was precipitated for examination by adding 0.1X volume of 3M Sodium acetate at pH5.2 and 2.5x volumes of 100% ethanol. This was then incubated on ice for 1h and the precipitated material pelleted by centrifugation 17,000g 20 minutes, decant supernatant and wash 2 x with 500μl 70% ethanol. The pellet was then air dried and resuspended in 100μl of nuclease free water. 25μl of this was then mixed 1:1 with denaturing loading dye (95% formamide, 0.1% xylene cyanol, 0.1% Bromphenol Blue, 20mM Tris-HCI pH 7.5) samples were boiled 95°C 2 minutes then to 4°C at 4°C/s cooling rate. The precipitated samples were then loaded onto a denaturing TBE-gel (12.5% polyacrylamide, 7M UREA). Run at 150V, 45minutes at room temperature. Once run the gel was washed 2 x in milliQ grade water to remove urea, then stained with 1 x SyBr gold (thermoscientific) DNA stain in 1 x TBE for 20minutes before washing again 2 x with water to remove staining solution.

The gels were then imaged using a Syngene InGenius3 gel doc system (Figure 3). It can be seen that when all reagents are present ligation occurs, but this is more efficient when using a heat step - although this is not required

for ligation to occur at reasonable levels as we have multiple copies of each DNA on a synthesis vesicle.

6) Release of chemical structure from encoding tag

The encoding oligonucleotide tag is removed by cleaving payload from the oligonucleotide.

Referring now to Figure 4, to show cleavage of the linker and release of the pharmacore oligonucleotides were made which incorporated a fluorophore (FITC, shown as F in Figure 4) 5' to the cleavable linker within the sequence of the cleavable oligonucleotide. A quencher molecule, BHQ-1, was then attached to the cleavable linker. This can be released by cleavage in the same manner as any chemical structure used in library generation. Light up of the oligonucleotide is seen when the quencher is removed from the cleavable linker.

Three different enzymes have been identified which cleave their associated linkers in a scarless manner (RNase A, RNase T1 A and RNase 1 A; labelled as enzymes A, B and C respectively in Figure 4). All 3 were tested with their compatriot linkers. In each case 10μl reactions were set up; 10mM Tris-HCl, 1mM EDTA, 150nM cleavable oligonucleotide 0.1μl of stock enzyme (RNase A = 10 mg/ml, RNase T1 = 10 mg/ml RNase 1 = 1.5 mg/ml) then to 10μl with nuclease free water. Samples were incubated for 15 minutes at 37 °C and then 10μL denaturing loading dye added (95% formamide, 0.1% xylene cyanol, 0.1% Bromphenol Blue, 20mM Tris-HCl pH 7.5). Samples were boiled for 2 minutes at 95°C and then rapidly cooled to 4°C at 4°C/s. These samples were then loaded onto a denaturing TBE-gel (12.5% polyacrylamide, 7M Urea). The gel was run at 150V for 75 minutes and the imaged using a Syngene Ingenius3 system exciting the FITC. Release of the quencher means the fluorophore can be seen and imaged as 'light up'. This is seen as appearance of yellow DNA band in the presence of enzyme. By staining with SyBr gold (Thermo) DNA can be seen and this is again quenched when quencher is present but the size shift corresponding to the change in mass can be seen as well as increased signal (grey scale image below fluorophore, Fig 4). In both cases we can see release only occurs in the presence of the enzymes and is rapid (<15 minutes). Additionally the enzymes are very active 10-fold serial dilutions of the enzymes were performed in 10mM Tris-HCl pH 7.5, 1mM EDTA and the reactions set up in the same manner. After dilution of the enzyme from $10^{-2}$ to $10^{-5}$, we can see activity at $10^{-4}$ for RNase A and $10^{-3}$ for RNase T1 and RNase 1. This means there is a large window of enzyme activity and that the process can occur in less than 30 minutes at $10^{-4}$ dilutions for multiple enzymes.

We also confirmed that the enzymes function in the droplet environment (see Figure 5). Results are shown for RNase A. 1ml of aqueous phase was set up using 10mM Tris-HCl, 1mM EDTA, 150nM cleavable oligonucleotide 10μl of stock RNase A (10mg/ml) then to 1ml with nuclease free water. Droplets of 100μM mean diameter were made using a 100μm etch depth chip with the aqueous phase as described above and the oil phase comprising of PSF-2 cST oil (Clearco Products Inc.) with 2 % w/v Gransurf G67 (Grant Industries Inc.) Droplets were incubated with and without enzyme at 37 °C for 15minutes and them imaged under a Floid fluorescence microscope illuminating at 482/18 nm and emission filters of 532/59 nm. It can be seen that light up occurs in droplets in the presence of enzyme and that the reaction is not affected by droplet environment.

7) Functionalisation of a cleavable chemical structure payload using Huisgen 1,3-dipolar cycloaddition and thiourethane formation

Two 100 μL portions of the beads functionalised with cleavable linker and core payload (hexynyl hydrodroxyprolinol) were washed three times with 100 mM MOPS pH 7.0 and resuspened in 100 μL of the same. To both portions 2 uL of a 10 mM stock of 5-FAM-azide (Jena Bioscience) in DMSO was added. One portion was kept as an unreacted negative control, to the other portion was added 5 μL of a 50 mM tris(3-hydroxypropyltriazolylmethyl)amine (THPTA, Sigma Aldrich), 2.5 μL of a 20 mM solution of copper (II) sulphate (Sigma Aldrich) and 100 μL of a 10 mM solution of sodium ascorbate (Sigma Aldrich). A further sample containing beads funtionalised with only the capture oligonucleotide (no cleavable linker or functional core) was also treated in the same way. The beads were placed on a rotator and left to react overnight, then the mix was spun down and washed with 1 x mL 100 mM MOPS buffer pH 7.0.

In order to confirm functionalisation, the functionalised payload was cleaved off the bead samples by suspending in 30 μL of NEB buffer 3.1, 10 μL BamH1 with 260 μL of nuclease free water. After incubation at 37 °C for one hour, the sample was split into 2 x 150 μL portions. 5 μL of RNase A was added, and the sample was incubated for a further one hour at 37 °C, then the beads were pelleted. The supernatant was transferred to a clean tube and 15 μL of 3M sodium acetate at pH 5.2 and 375 μL of 100 % ethanol were added. The sample was left to precipitate overnight at -20 °C, then pelleted, washed with 500 uL of 70 % ethanol and air dried for 20 mins. The pellet was resuspened in 20 uL of nuclease free water, 20 μL of denaturing loading dye was added (95% formamide, 0.1% xylene cyanol, 0.1% Bromphenol Blue, 20mM Tris-HCl pH 7.5). The sample was heated for five minutes at 95 °C then cooled to 4 °C. The samples were loaded onto a TBE urea gel and ran at 100 V for 75 mins.

As shown in Figure 6, a free dyed component is visible in the sample treated with both the ligated cleavable linker, functional core and dye, indicating a functionalised and released core rather than the presence of free 5-FAM

azide, which would be present in the unligated sample.

Separately, two further 100 μL portions of the functionalised beads were washed three times with dry acetonitrile (MeCN, Sigma Aldrich). One portion was suspended in 1 mM fluorescein isothiocyanate isomer 1 (Sigma Aldrich) in MeCN, along with 0.6 μL of a 10 mg/mL solution of triethylene diamine (Sigma Aldrich) in MeCN. After rotating overnight at 37 °C, the beads were washed 3x with MeCN, then 3x with 100 mM sodium hydrogen carbonate buffer, pH 7.5 In order to confirm functionalisation, the samples were cleaved enzymatically as described above and run on a TBE gel. The gel was imaged using a Syngene InGenius3 gel doc system to observe the fluorophore, then stained with 50 μL of 1xSybr gold in TBE. A second image was then recorded.

As shown in Figure 7, an intense signal is seen in the digested sample treated with RNase A compared to the no dye control, indicating functionalisation of the cleaved compound.

**8)** Construction of a Functional Library of 100 members on Beads with Tagging using a Split and Pool Methodology

A stock of azide functionalised beads was functionalised with capture oligo as described in section 3, example 4. This stock of beads was then ligated with a cleavable oligo bearing a cleavable payload (5'ATTCCCCTAGCTATGCAAGTrGrArGRrArArGrUX3', where X = 3'-(O-Propargyl)-adenosine) (ADTBio), using the same technique as described in Example 4 (section 4). The number of beads was measured to be 5.6 x $10^6$ beads/mL by haemocytometer. 200 μL of this stock was added to ten separate wells of a deep 96 well plate (Fisher). In the following description, all stock solutions were made up in 100 mM MOPS pH 7.0, unless otherwise stated. The plate was centrifuged and 140 μL of 100 mM MOPS pH 7.0, 100 mM, was added to each occupied well, followed by 20 μL of a 50 mM stock of Tris(3-hydroxypropyltriazolylmethyl)amine (Sigma-Aldrich) and 10 μL of a 20 mM stock of 20 mM copper (II) sulfate (Sigma-Aldrich).

One of ten individually selected azides (10 μL of a 50 mM DMSO, Enamine) was added to each separate occupied well. 20 μL of a 100 mM stock of sodium ascorbate (Sigma-Aldrich) was added and the plate was sealed using a peaceable sealing cap (Fisher). The plate was incubated at room temperature with shaking for 18 hours. The plate was then centrifuged and each occupied well was washed with 3 x 1 mL of nuclease free water (NFW, Fisher), then resuspended in 250 μL of Quick Ligase Buffer 2x (NEB).

200 μL of a 50 mM stock of a coding oligo comprising the sequence GAAGGGTCGACTCCGXXXXXXXXXXGATGGGCATCATCCT, where XXXXXXXXX = represents a random sequence of nucleotides (A, C G or T) chosen from a selection of 20 unique coding oligos, each from Integrated DNA Technologies (IDT) was added to each well. 50 μL of a 100 uM splinting oligo (CTT AGT CGA CCC GGC AGG ATG ATG CCC AT/3ddC/, /3ddC = dideoxycytidine, IDT) was added along with 2.5 μL of Quick Ligase (NEB). The plate was sealed and incubated with shaking at 37 °C.

Each individual population of beads across 10 wells was now encoded with a unique DNA sequence associated with a specific azide, enabling the building blocks used to be tracked after screening to determine the structure of any compound. After two hours incubation, each well was washed with 3 x 1 mL of NFW and resuspended in 200 μL of 200 mM sodium acetate, pH 5.5. The contents of each well was then combined together as a 2 mL mixed pool in a 15 mL centrifuge tube and thoroughly mixed by vortexing, before being split in 200 μL portions in 10 separate wells on a new deep 96 well plate. The plate was centrifuged and the beads resuspened in 190 μL of 200 mM sodium acetate pH 5.5. A stock of one of ten individually selected aldehydes (83 μL of 50 mM DMSO stock, Enamine) was added to the individual wells. 26 μL of a 10 mg/mL stock of sodium cyanoborohydride in 200 mM sodium acetate pH 5.5 (sigma Aldrich) was then added to each occupied well. The plate was sealed and incubated with shaking for 18 hours at room temperature. All wells were washed 3 x 1 mL with nuclease free water, then resuspened in 250 μL of quick ligase buffer, and each well was ligated with a unique coding oligo using the same process as previous described for the azide block. Each well was then washed with 3 x 1 mL of NFW and resuspened in 200 μL nuclease free water, before being pooled into a 2 mL stock in a 15 mL centrifuge tube. The beads were pelleted and resuspened in 200 μL 2x Quick Ligase buffer. 200 μL of a 100 uM stock of oligo with the sequence CGGGTCGACTTCGGTTAGACTTTCGGACCTGATGGGCATCATCCT was added, along with 10 uL of Quick Ligase (New England Biolabs). The beads were then incubated at 37 °C for two hours with rotation, then washed 3 x with 1 mL of 10 mM Tris HCl + 1 mM EDTA pH 7.5. The beads were resuspened in 1 mL of the Tris buffer then stored at 4 °C until needed.

## Claims

1. A method for screening an encoded chemical library, which library comprises a number *n* of clonal populations of different chemical structures each releasably linked to an encoding tag by a cleavable linker via a bead, and each clonal population being confined to *n* discrete library microcompartments, the method comprising the steps of: (a) providing said library of tagged chemical structures; (b) placing each of the library microcompartments into discrete screening microdroplets, which microdroplets comprise a gelling system; (c) releasing each chemical structure from

its tag to produce a plurality of free, tagless chemical structures (TCSs); (d) phenotypically screening the TCSs by contacting them with an assay system comprising a live target cell under conditions whereby a spatial association between each TCS and its tag is maintained, to produce a plurality of different screened TCSs each spatially associated with its tag; and (e) identifying a screened TCS by decoding a tag that is spatially associated therewith, wherein the gelling system of the microdroplets is gelled prior to the identifying step (e) such that the library microcompartment becomes fixed within the gelled microdroplet.

**2.** The method of claim 1, wherein the encoding tag comprises a nucleic acid, the method being for screening a nucleic acid-encoded chemical library.

**3.** The method of any one of the preceding claims, wherein the chemical structures are releasably linked to the encoding tag by a cleavable linker, optionally wherein the cleavable linker comprises a linker selected from: enzymatically cleavable linkers; nucleophile/base-sensitive linkers; reduction sensitive linkers; photocleavable linkers; electrophile/acid-sensitive linkers; metal-assisted cleavage-sensitive linkers; oxidation-sensitive linkers; and combinations of two or more of the foregoing.

**4.** The method of any one of the preceding claims, wherein the chemical structures are releasably linked to the encoding tag by a self-immolative linker comprising a peptide or non-peptide enzymatically cleavage moiety and a self-immolative moiety (SIM).

**5.** The method of any one of the preceding claims, wherein a single bead is loaded with a plurality of chemical structures, for example such that the ratio of encoding tag(s) to linked chemical structures is 1:10 to 1:1000.

**6.** The method of any one of the preceding claims, wherein the tags of step (d) are functionally or physically partitioned from the assay system.

**7.** The method of any one of claims 2-6, wherein the encoding nucleic acid tag is a template for the chemical structure.

**8.** The method of claim 7, wherein step (a) comprises the step of nucleic acid-templated, for example DNA-templated, synthesis of the chemical structures.

**9.** The method of any one of claims 1-8, wherein the library comprises a clonal population of chemical structures and step (a) comprises the step of releasably linking an encoding tag to each of the chemical structures within said clonal population.

**10.** Use of an encoded chemical library in a method according to any one of the preceding claims, which library comprises a number *n* of clonal populations of chemical structures each releasably linked to an encoding tag, each clonal population being confined to *n* discrete library microcompartments.

**11.** Use according to claim 10, wherein the chemical structures are linked to the encoding tags by a cleavable linker as defined in claim 4.

**12.** Use according to claim 10 or claim 11, wherein the chemical structures are contained within the microcompartments together with encoding tags but are not covalently linked to the encoding tags.

**13.** Use of an assay composition in a method according to any one of claims 1-9 comprising the library of any one of claims 10-12 in which the chemical structures contained within the microcompartments are in contact with an assay system.

**14.** Use of a nucleic encoded chemical library reactor in a method according to any one of claims 1-9 comprising a microcompartment containing: (a) a clonal population of encoding nucleic acid template molecules; and (b) a plurality of chemical substructures, wherein the substructures are adapted for nucleic acid-templated assembly to form encoding nucleic-acid-tagged chemical structures in which the encoding tags are releasably linked to the chemical structures.

## Patentansprüche

1. Verfahren zum Screenen einer kodierten chemischen Bibliothek, wobei die Bibliothek eine Anzahl *n* klonaler Populationen unterschiedlicher chemischer Strukturen umfasst, von denen jede durch einen spaltbaren Linker über ein Kügelchen lösbar mit einer kodierenden Markierung verknüpft ist, und wobei jede klonale Population auf *n* einzelne Bibliotheksmikrokammern beschränkt ist, wobei das Verfahren die folgenden Schritte umfasst: (a) Bereitstellen der Bibliothek markierter chemischer Strukturen; (b) Platzieren jeder der Bibliotheksmikrokammern in einzelnen Screening-Mikrotröpfchen, wobei die Mikrotröpfchen ein Gelierungssystem umfassen; (c) Lösen jeder chemischen Struktur von ihrer Markierung, um eine Vielzahl von freien, markierungslosen chemischen Strukturen (TCSs) zu erzeugen; (d) phänotypisches Screening der TCSs durch Kontaktieren mit einem Assay-System, das eine lebende Zielzelle umfasst, unter Bedingungen, bei denen eine räumliche Assoziation zwischen jedem TCS und seiner Markierung aufrechterhalten wird, um eine Vielzahl unterschiedlicher gescreenter TCSs zu erzeugen, die jeweils mit seiner Markierung räumlich assoziiert sind; und (e) Identifizieren eines gescreenten TCS durch Dekodieren einer Markierung, die räumlich damit assoziiert ist, wobei das Gelierungssystem der Mikrotröpfchen vor dem Identifizierungsschritt (e) geliert wird, sodass die Bibliotheksmikrokammer innerhalb des gelierten Mikrotröpfchens fixiert wird.

2. Verfahren nach Anspruch 1, wobei die kodierende Markierung eine Nukleinsäure umfasst, wobei das Verfahren zum Screenen einer Nukleinsäure-kodierten chemischen Bibliothek ist.

3. Verfahren nach einem der vorhergehenden Ansprüche, wobei die chemischen Strukturen durch einen spaltbaren Linker lösbar mit der kodierenden Markierung verknüpft sind, wobei der spaltbare Linker optional einen Linker umfasst, ausgewählt aus: enzymatisch spaltbaren Linkern; nukleophilen/basenempfindlichen Linkern; reduktionsempfindlichen Linkern; photospaltbaren Linkern; elektrophilen/säureempfindlichen Linkern; metallunterstützten spaltungsempfindlichen Linkern; oxidationsempfindlichen Linkern; und Kombinationen von zwei oder mehr des Vorhergehenden.

4. Verfahren nach einem der vorhergehenden Ansprüche, wobei die chemischen Strukturen durch einen selbstzerlegenden Linker, der eine enzymatisch spaltende Peptid- oder Nicht-Peptid-Gruppierung und eine selbstzerlegende Gruppierung (SIM) umfasst, lösbar mit der kodierenden Markierung verknüpft sind.

5. Verfahren nach einem der vorhergehenden Ansprüche, wobei ein einzelnes Kügelchen mit einer Vielzahl von chemischen Strukturen beladen wird, beispielsweise derart, dass das Verhältnis von kodierenden Markierung(en) zu verknüpften chemischen Strukturen 1:10 bis 1:1000 ist.

6. Verfahren nach einem der vorhergehenden Ansprüche, wobei die Markierungen von Schritt (d) funktionell oder physikalisch von dem Assay-System abgetrennt sind.

7. Verfahren nach einem der Ansprüche 2-6, wobei die kodierende Nukleinsäuremarkierung eine Vorlage für die chemische Struktur ist.

8. Verfahren nach Anspruch 7, wobei Schritt (a) den Schritt der Synthese mit Nukleinsäurevorlage, beispielsweise DNA-Vorlage, der chemischen Strukturen umfasst.

9. Verfahren nach einem der Ansprüche 1-8, wobei die Bibliothek eine klonale Population chemischer Strukturen umfasst und Schritt (a) den Schritt des lösbaren Verknüpfens einer kodierenden Markierung mit jeder der chemischen Strukturen innerhalb der klonalen Population umfasst.

10. Verwendung einer kodierten chemischen Bibliothek in einem Verfahren nach einem der vorhergehenden Ansprüche, wobei die Bibliothek eine Anzahl *n* klonaler Populationen chemischer Strukturen umfasst, von denen jede lösbar mit einer kodierenden Markierung verknüpft ist, wobei jede klonale Population auf *n* einzelne Bibliotheksmikrokammern beschränkt ist.

11. Verwendung nach Anspruch 10, wobei die chemischen Strukturen mit den kodierenden Markierungen durch einen spaltbaren Linker wie in Anspruch 4 definiert verknüpft sind.

12. Verwendung nach Anspruch 10 oder Anspruch 11, wobei die chemischen Strukturen in den Mikrokammern zusammen mit kodierenden Markierungen enthalten sind, aber nicht kovalent mit den kodierenden Markierungen verknüpft sind.

13. Verwendung einer Assay-Zusammensetzung in einem Verfahren nach einem der Ansprüche 1-9, umfassend die Bibliothek nach einem der Ansprüche 10-12, wobei die chemischen Strukturen, die in den Mikrokammern enthalten sind, mit einem Assay-System in Kontakt sind.

14. Verwendung eines Nukleinsäure-kodierten chemischen Bibliotheksreaktors in einem Verfahren nach einem der Ansprüche 1-9, umfassend eine Mikrokammer, enthaltend: (a) eine klonale Population kodierender Nukleinsäurevorlagenmoleküle; und (b) eine Vielzahl von chemischen Unterstrukturen, wobei die Unterstrukturen für Anordnung mit Nukleinsäurevorlage ausgelegt sind, um kodierende nukleinsäuremarkierte chemische Strukturen zu bilden, in denen die kodierenden Markierungen lösbar mit den chemischen Strukturen verknüpft sind.

## Revendications

1. Procédé destiné à cribler une chimiothèque codée, laquelle chimiothèque comprend un nombre n de populations clonales de structures chimiques différentes, chacune liée de manière détachable à une étiquette de codage par un segment de liaison clivable via une bille, et chaque population clonale étant confinée dans n microcompartiments de bibliothèque individuels, le procédé comprenant les étapes suivantes : (a) fourniture de ladite bibliothèque de structures chimiques étiquetées ; (b) mise en place de chacun des microcompartiments de bibliothèque dans des microgouttelettes de criblage individuelles, lesquelles microgouttelettes comprennent un système de gélification ; (c) libération de chaque structure chimique de son étiquette pour produire une pluralité de structures chimiques sans étiquette, libres (SCS) ; (d) criblage phénotypique des SCS en les mettant en contact avec un système de dosage comprenant une cellule cible vivante dans des conditions dans lesquelles une association spatiale entre chaque SCS et son étiquette est maintenue, pour produire une pluralité de SCS criblées différentes, chacune associée spatialement à son étiquette ; et (e) identification d'une SCS criblée en décodant une étiquette qui y est associée spatialement, dans lequel le système de gélification des microgouttelettes est gélifié avant l'étape d'identification (e) de sorte que le microcompartiment de bibliothèque devient fixe au sein de la microgouttelette gélifiée.

2. Procédé selon la revendication 1, dans lequel l'étiquette de codage comprend un acide nucléique, le procédé étant destiné à cribler une chimiothèque codée par acide nucléique.

3. Procédé selon l'une quelconque des revendications précédentes, dans lequel les structures chimiques sont liées de manière détachable à l'étiquette de codage par un segment de liaison clivable, facultativement dans lequel le segment de liaison clivable comprend un segment de liaison choisi parmi : des segments de liaison clivables de manière enzymatique ; des segments de liaison nucléophiles/sensibles aux bases ; des segments de liaison sensibles à la réduction ; des segments de liaison photoclivables ; des segments de liaison électrophiles/sensibles aux acides ; des segments de liaison sensibles au clivage assisté par des métaux ; des segments de liaison sensibles à l'oxydation ; et des combinaisons de deux des éléments précédents ou plus.

4. Procédé selon l'une quelconque des revendications précédentes, dans lequel les structures chimiques sont liées de manière détachable à l'étiquette de codage par un segment de liaison auto-immolable comprenant un groupement de clivage enzymatique peptidique ou non peptidique et un groupement auto-immolable (GAI).

5. Procédé selon l'une quelconque des revendications précédentes, dans lequel une bille unique est chargée avec une pluralité de structures chimiques, par exemple de sorte que le rapport d'étiquette(s) de codage et de structures chimiques liées va de 1/10 à 1/1 000.

6. Procédé selon l'une quelconque des revendications précédentes, dans lequel les étiquettes de l'étape (d) sont partitionnées de manière fonctionnelle ou physique à partir du système de dosage.

7. Procédé selon l'une quelconque des revendications 2 à 6, dans lequel l'étiquette d'acide nucléique de codage est une matrice pour la structure chimique.

8. Procédé selon la revendication 7, dans lequel l'étape (a) comprend l'étape de synthèse à matrice d'acide nucléique, par exemple à matrice d'ADN, des structures chimiques.

9. Procédé selon l'une quelconque des revendications 1 à 8, dans lequel la bibliothèque comprend une population clonale de structures chimiques et l'étape (a) comprend l'étape de liaison détachable d'une étiquette de codage à chacune des structures chimiques au sein de ladite population clonale.

**10.** Utilisation d'une chimiothèque codée dans un procédé selon l'une quelconque des revendications précédentes, laquelle bibliothèque comprend un nombre n de populations clonales de structures chimiques, chacune liée de manière détachable à une étiquette de codage, chaque population clonale étant confinée dans n microcompartiments de bibliothèque individuels.

**11.** Utilisation selon la revendication 10, dans laquelle les structures chimiques sont liées aux étiquettes de codage par un segment de liaison clivable tel que défini dans la revendication 4.

**12.** Utilisation selon la revendication 10 ou la revendication 11, dans laquelle les structures chimiques sont contenues au sein des microcompartiments conjointement avec des étiquettes de codage mais ne sont pas liées par covalence aux étiquettes de codage.

**13.** Utilisation d'une composition de dosage dans un procédé selon l'une quelconque des revendications 1 à 9 comprenant la bibliothèque selon l'une quelconque des revendications 10 à 12 dans laquelle les structures chimiques contenues au sein des microcompartiments sont en contact avec un système de dosage.

**14.** Utilisation d'un réacteur de chimiothèque codée par acide nucléique dans un procédé selon l'une quelconque des revendications 1 à 9 comprenant un microcompartiment contenant : (a) une population clonale de molécules de matrice d'acide nucléique de codage ; et (b) une pluralité de sous-structures chimiques, dans laquelle les sous-structures sont conçues pour l'assemblage par matrice d'acide nucléique pour former des structures chimiques étiquetées par acide nucléique de codage dans lesquelles les étiquettes de codage sont liées de manière détachable aux structures chimiques.

FIGURE 1

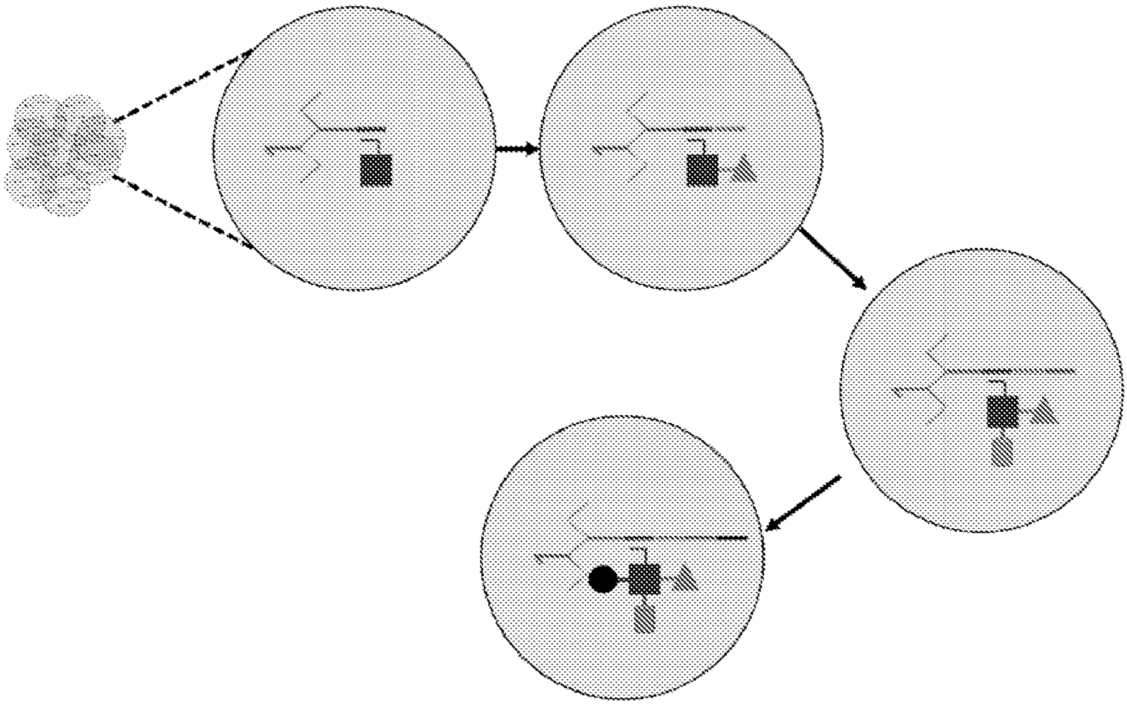

FIGURE 2

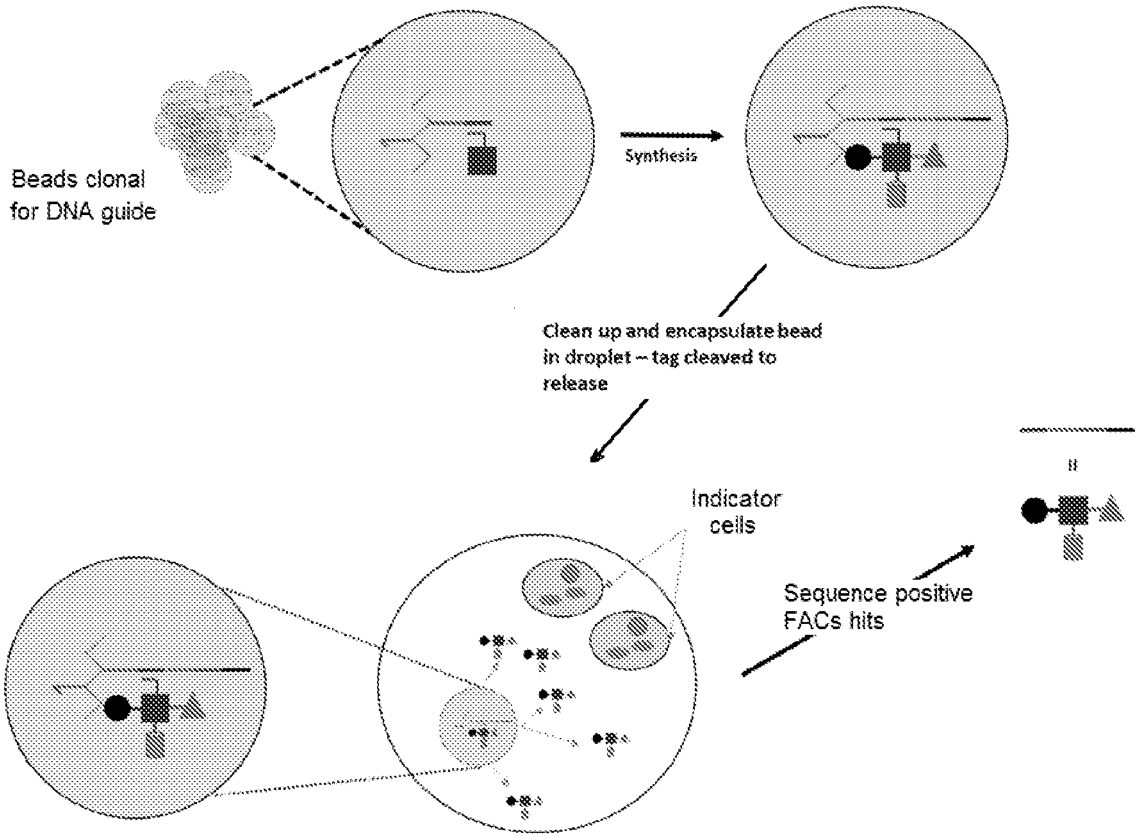
Beads clonal
for DNA guide
Synthesis
Clean up and encapsulate bead
in droplet – tag cleaved to
release
Indicator
cells
Sequence positive
FACs hits

FIGURE 3

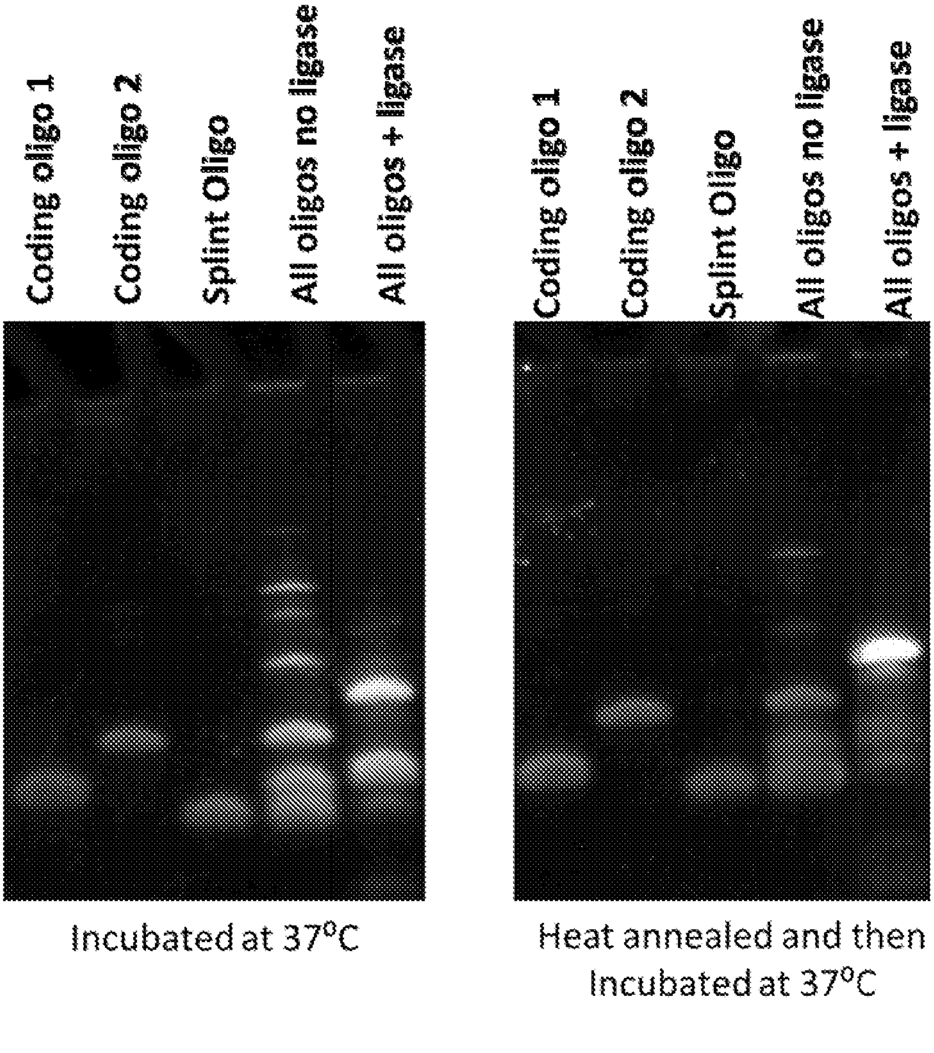
Coding oligo 1
Coding oligo 2
Splint Oligo
All oligos no ligase
All oligos + ligase
Incubated at 37°C
Coding oligo 1
Coding oligo 2
Splint Oligo
All oligos no ligase
All oligos + ligase
Heat annealed and then
Incubated at 37°C

FIGURE 4

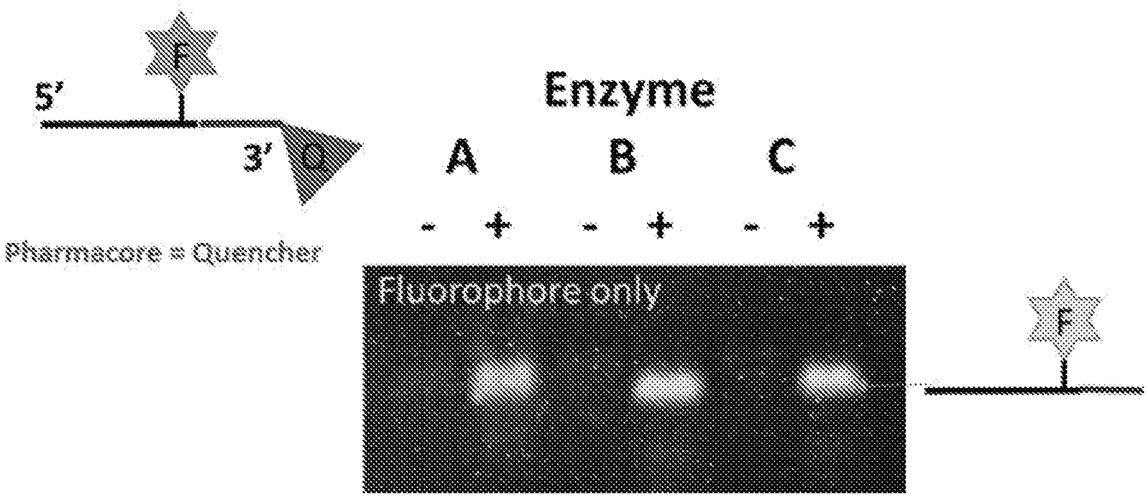
5'
F
3'
Q
Pharmacore = Quencher
Enzyme
A
B
C
- + - + - +
Fluorophore only
F

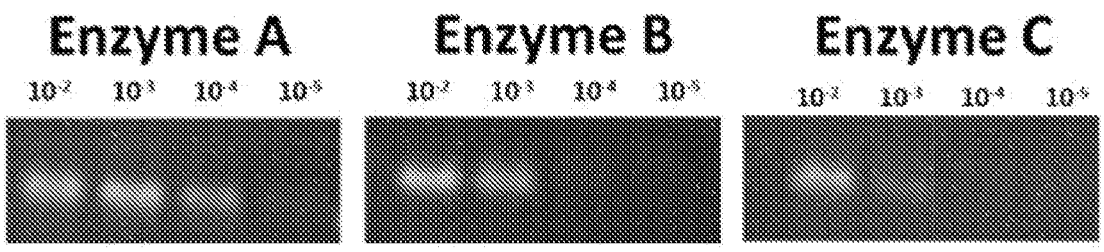
Enzyme A
Enzyme B
Enzyme C

FIGURE 5

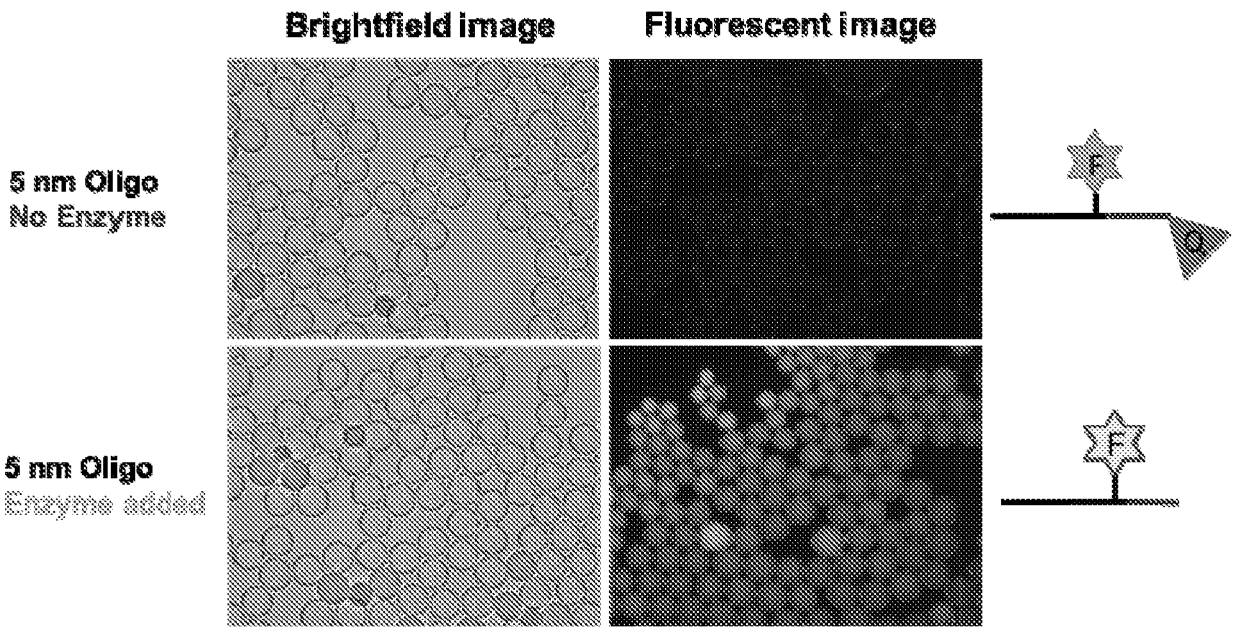
Brightfield image
Fluorescent image
5 nm Oligo
No Enzyme
5 nm Oligo
Enzyme added
F
Q
F

FIGURE 6

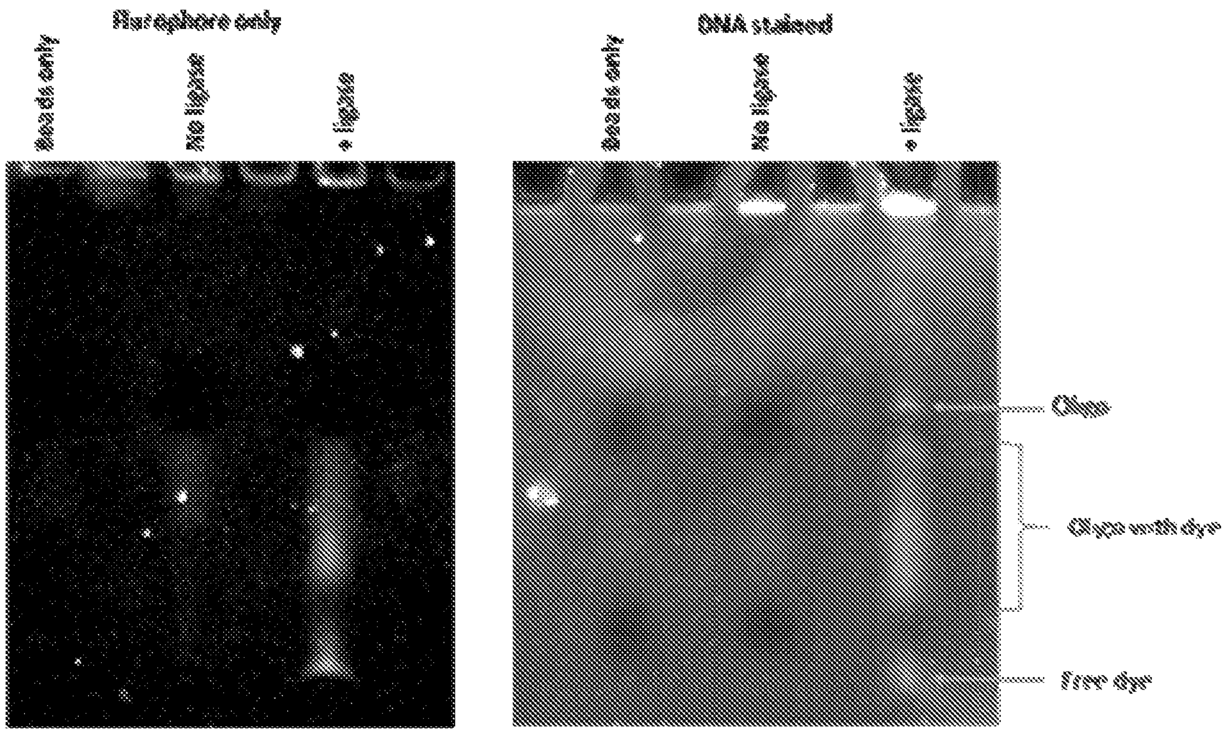
Fluorophore only
Beads only
No ligase
+ ligase
DNA stained
Beads only
No ligase
+ ligase
Oligo
Oligo with dye
Free dye

FIGURE 7

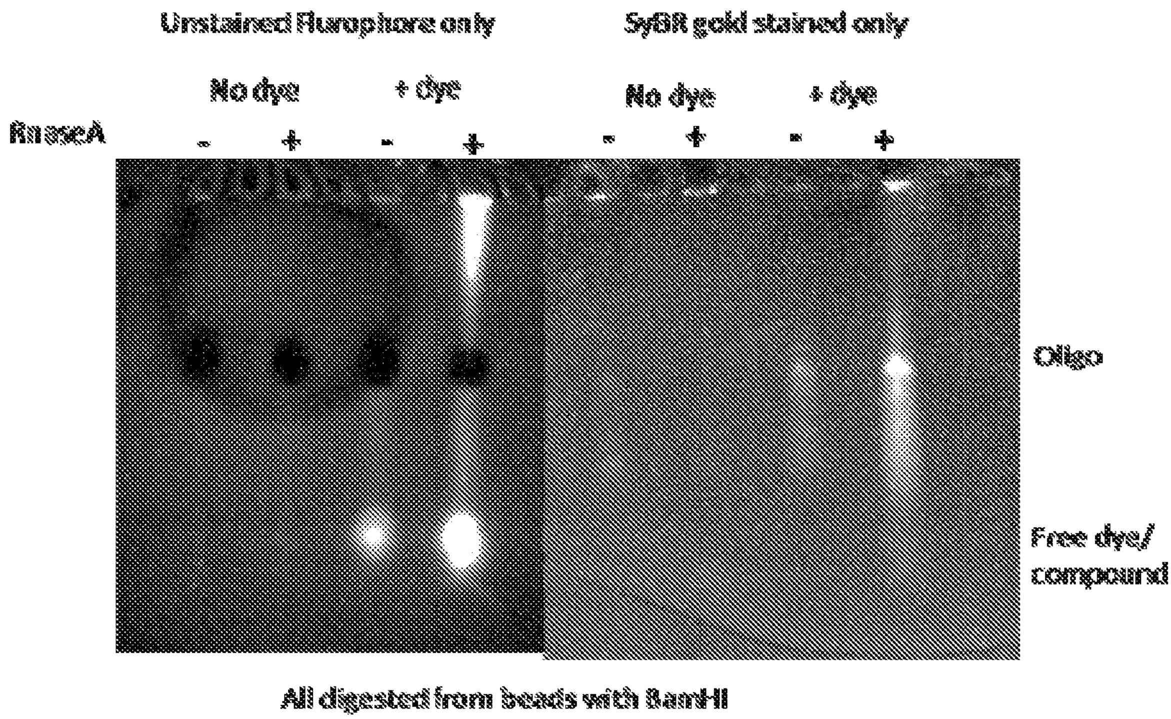
Unstained Flurophore only
SyBR gold stained only
No dye
+ dye
No dye
+ dye
RnaseA
- + - + - + - +
Oligo
Free dye/
compound
All digested from beads with BamHI

FIGURE 8

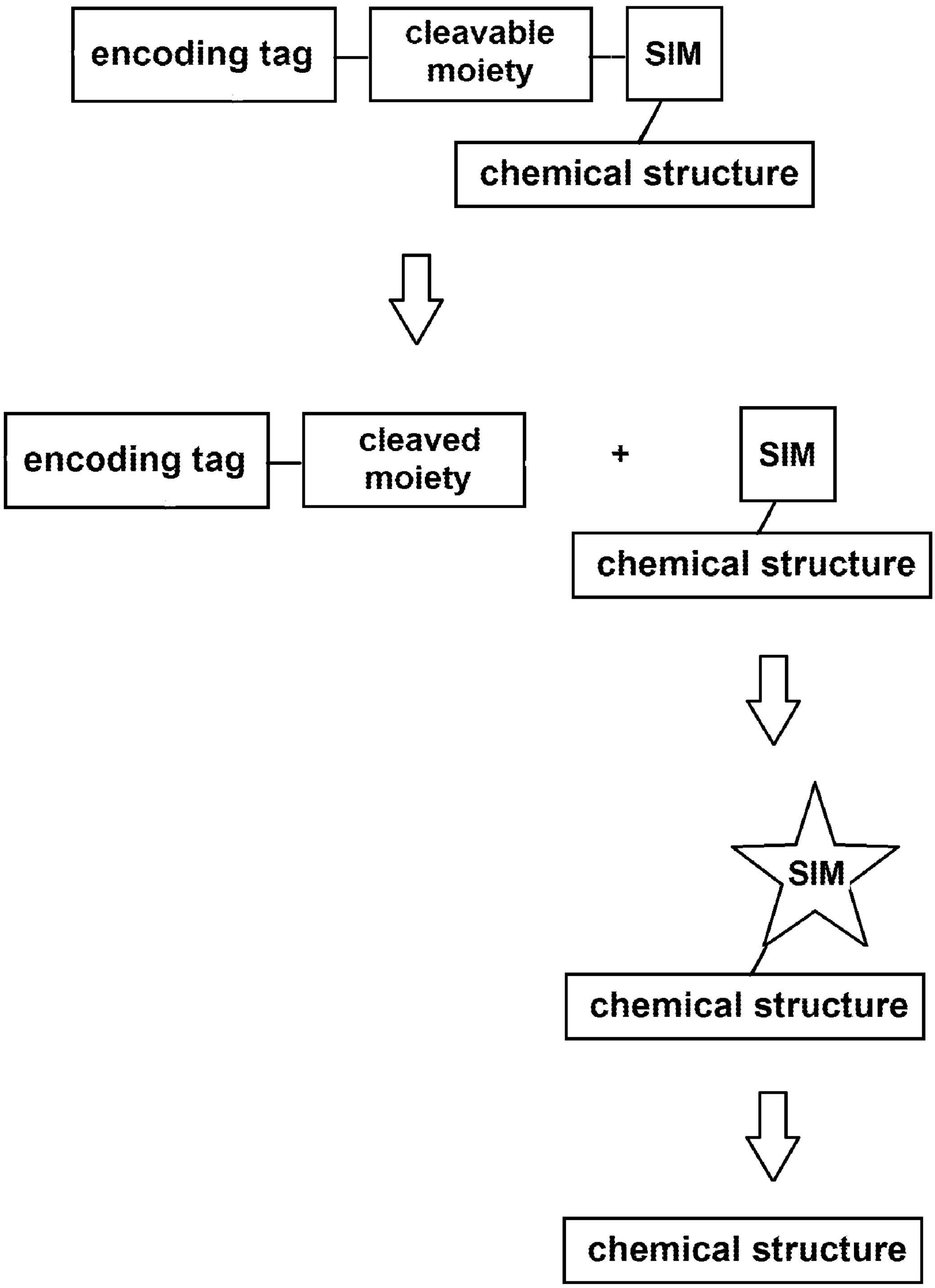
encoding tag
cleavable moiety
SIM
chemical structure
encoding tag
cleaved moiety
+
SIM
chemical structure
SIM
chemical structure
chemical structure

FIGURE 9

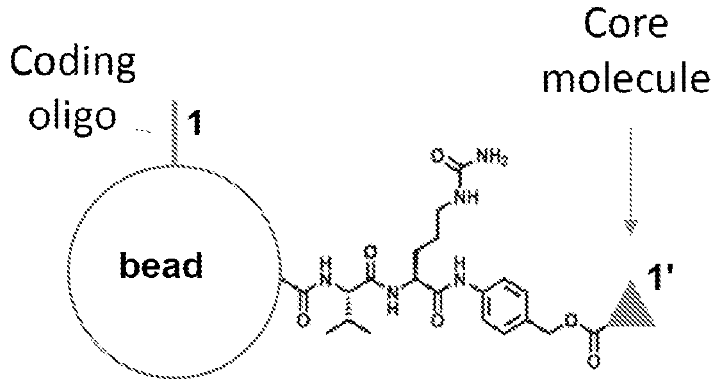


Coding oligo/payload ratio 1:10-1000

Split and Pool functionalisation

Encode each new building block

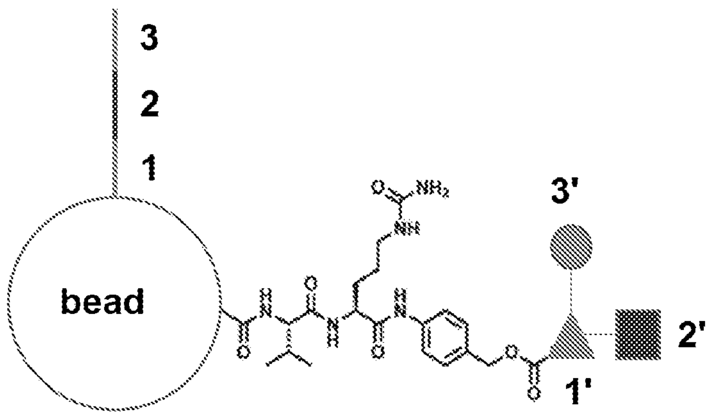

FIGURE 10

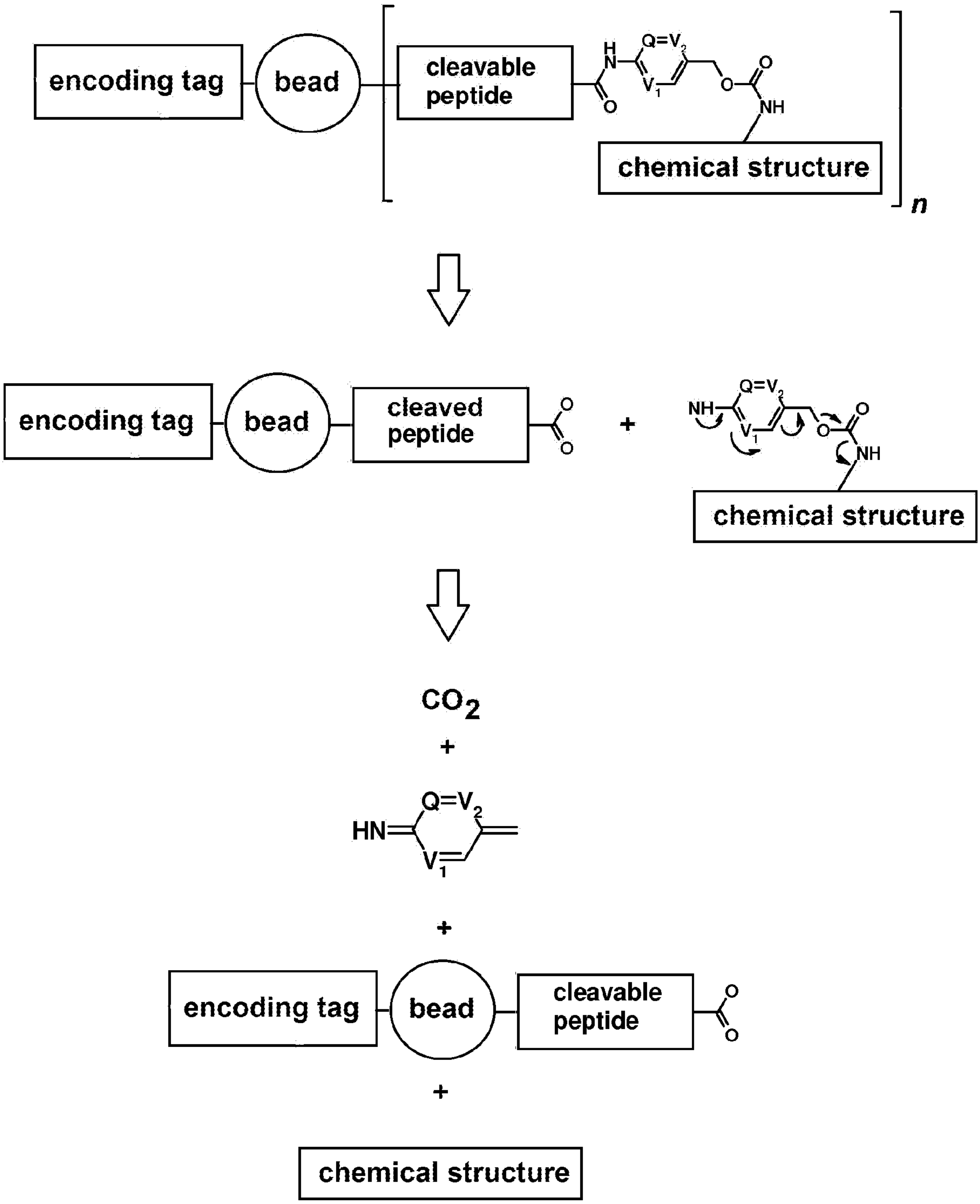
encoding tag
bead
cleavable peptide
chemical structure
encoding tag
bead
cleaved peptide
chemical structure
encoding tag
bead
cleavable peptide
chemical structure

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006048025 A
- WO 2015021080 A
- WO 9409817 A
- WO 2010009365 A
- WO 2006040551 A
- WO 2006040554 A
- WO 2004002627 A
- WO 2004091763 A
- WO 2005021151 A
- WO 2006096571 A
- WO 2007089541 A
- WO 2007081385 A
- WO 2008063227 A
- WO 2008021123 A
- WO 9623810 A
- WO 2017089894 A
- WO 2016146638 A
- US 2010273843 A
- WO 2005112919 A
- WO 02083180 A
- WO 2007011968 A
- US 20170189542 A


### Non-patent literature cited in the description


- **CLAUSELL-TORMOS et al.** *Chemistry & Biology,* 2008, vol. 15, 427-437
- **BRENNER ; LERNER.** *Proc. Natl. Acad. Sci. USA.,* 1992, vol. 89, 5381-5383
- **MANNOCCI et al.** *Chem. Commun.,* 2011, vol. 47, 12747-12753
- **KLEINER et al.** *Chem Soc Rev.,* 2011, vol. 40 (12), 5707-5717
- **MULLARD.** *Nature,* 2016, vol. 530, 367-369
- **SHEMBEKAR et al.** *Lab Chip,* 2016, vol. 16, 1314-1331
- **LUNDBERG et al.** *Nucleic acids symposium,* 2008, vol. 52, 683-684
- **ROTHEMUND.** *Nature,* 2006, vol. 440, 297-302
- **ODOM et al.** *J. AM. CHEM. SOC.,* 2002, vol. 124, 12112-12113
- **BERNATH et al.** *Analytical Biochemistry,* 2004, vol. 325, 151-157
- **HOLTZE ; WEITZ.** *Lab Chip,* 2008, vol. 8 (10), 1632-1639
- **HOLTZE et al.** *Lab Chip,* 2008, vol. 8 (10), 1632-1639
- **KREUTZ et al.** *J Am Chem Soc.,* 2009, vol. 131 (17), 6042-6043
- **THEBERGE et al.** *Chem. Commun.,* 2009, 6225-6227
- **CLAUSELL-TORMOS et al.** *Chem Biol.,* 2008, vol. 15 (5), 427-37
- **ANNA et al.** *Appl. Phys. Lett.,* 2003, vol. 82 (3), 364-366
- **EASTBURN et al.** Picoinjection Enables Digital Detection of RNA with Droplet RT-PCR. *PLoS ONE,* 2013, vol. 8 (4), e62961
- **TAN ; TAKEUCHI.** *Lab Chip,* 2006, vol. 6 (6), 757-63
- **SIMON ; LEE.** Microdroplet Technology. *Integrated Analytical Systems,* 2012, 23-50
- **BARET et al.** *Lab Chip,* 2009, vol. 9, 1850-1858
- **CHALFIE et al.** *Science,* 1994, vol. 263, 802-805
- **HEIM et al.** *Nature,* 1995, vol. 373, 663-4
- **LERICHE et al.** *Bioorganic & Medicinal Chemistry,* 2012, vol. 20 (2), 571-582
- **DE GROOT et al.** *J. Med. Chem.,* 1999, vol. 42, 5277
- **DE GROOT et al.** *J Org. Chem.,* 2000, vol. 43, 3093
- **DE GROOT et al.** *J Med. Chem.,* 2001, vol. 66, 8815
- **CARL et al.** *J Med. Chem. Lett.,* 1981, vol. 24, 479
- **STUDER et al.** *Bioconjugate Chem.,* 1992, vol. 3 (5), 424-429
- **CARL et al.** *J. Med. Chem.,* 1981, vol. 24 (5), 479-480
- **DUBOWCHIK et al.** *Bioorg & Med. Chern. Lett.,* 1998, vol. 8, 3347
- **MCCOMBS ; OWEN.** Antibody Drug Conjugates: Design and Selection of Linker. *Payload and Conjugation Chemistry The AAPS Journal,* 2015, vol. 17 (2), 339-351
- **HAY et al.** *Bioorg. Med. Chem. Lett.,* 1999, vol. 9, 2237

- **RODRIGUES et al.** *Chemistry Biology,* 1995, vol. 2, 223
- **STORM et al.** *J. Amer. Chem. Soc.,* 1972, vol. 94, 5815
- **AMSBERRY et al.** *J. Org. Chem.,* 1990, vol. 55, 5867
- **MANNOCCI et al.** *Proc. Natl. Acad. Sci.,* 2008, vol. 105, 17670-17675